(19) Europäisches Patentamt
European Patent Office
Office européen des brevets

(11) Numéro de publication: **0 282 412 B1**

# (12) FASCICULE DE BREVET EUROPEEN

(45) Date de publication de fascicule du brevet: **29.07.92**

(51) Int. Cl.[5]: **G01N 33/92**, G01N 33/559, G01N 33/561

(21) Numéro de dépôt: **88400555.4**

(22) Date de dépôt: **09.03.88**

(54) **Nouveau procédé de dosage simultané d'au moins deux sous ensembles d'apolipoprotéines contenues dans un milieu biologique.**

(30) Priorité: **10.03.87 FR 8703259**

(43) Date de publication de la demande:
**14.09.88 Bulletin 88/37**

(45) Mention de la délivrance du brevet:
**29.07.92 Bulletin 92/31**

(84) Etats contractants désignés:
**AT BE CH DE ES GB GR IT LI LU NL SE**

(56) Documents cités:
**EP-A- 0 129 696**
**WO-A-86/04144**

**CLINICAL CHEMISTRY, vol.28, no.1, janvier 1982, Easton, PA (US); J.-C.FRUCHART et al., pp.59-62**

**BIOLOGICAL ABSTRACTS, vol.70, no.4, 1980, Philadelphia, PA (US); D.BIOU et al., p.2202, no.20955**

(73) Titulaire: **LABORATOIRES SEBIA**
**23 rue Maximilien Robespierre**
**F-92130 Issy-Les-Moulineaux(FR)**

(72) Inventeur: **Bellon, Franck**
**Appt. 2512, Tour Eve**
**F-92800 Puteaux(FR)**
Inventeur: **Fruchart, Jean-Charles**
**23 Domaine de la Hollande, Ennetières en Weppes**
**F-59390 Haubourdin(FR)**
Inventeur: **Barouh, Guy**
**37 Boulevard Murat**
**F-75016 Paris(FR)**

(74) Mandataire: **Grosset-Fournier, Chantal Catherine et al**
**SC Ernest Gutmann/Yves Plasseraud 67 boulevard Haussmann**
**F-75008 Paris(FR)**

EP 0 282 412 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

L'invention à pour objet un nouveau procédé pour déterminer simultanément le contenu de chacun de deux ou plusieurs groupes de particules distinctes et indépendantes dans un mélange de particules. L'invention à également pour objet un kit pour le mise en oeuvre de de ce procédé.

L'invention a également pour objet un nouveau procédé de dosage simultané d'au moins deux sous-ensembles d'apolipoprotéines contenues dans un milieu biologique.

L'invention a également pour objet de nouveaux supports plans (plaques ou films) prêts à l'utilisation, et permettant la mise en oeuvre du procédé de l'invention.

L'invention vise également des kits ou nécessaires de dosage pour la mise en oeuvre du procédé de l'invention.

Enfin, l'invention vise également un procédé de dépistage in vitro de risques d'athérosclérose corrélés à des teneurs en apolipoprotéines déterminées, portées par des particules lipoprotéiques, et situées à l'extérieur du domaine délimité par les valeurs extrêmes des susdites apolipoprotéines correspondant à l'état physiologique d'un individu sain, en mettant en oeuvre le procédé de dosage selon l'invention.

Il est aujourd'hui parfaitement démontré que les dyslipoprotéinémies constituent le facteur prédisposant majeur pour le développement et la progression des lésions artérielles (Lipids Research Clinic Program. The lipid research clinics coronary primary prevention trial results. I. Reduction in incidence of coronary heart disease. II. The relationship of reduction in incidence of coronary heart disease to cholesterol lowering. JAMA, 1984, 251:351-374 ; Ducimetière p., Richard J.L., Claude J.R., Warnet J.M. Les cardiopathies ischémiques. Incidence et facteurs de risque. In : L'Etude Prospective Parisienne, Paris, INSERM 1981 : Fruchart J.C., Bertrand M., Parra H., Gentilini J.L., Boniface B. , Lipoprotéines et apolipoprotéines plasmatiques. Intérêt de leur dosage dans le dépistage de l'athérosclérose coronarienne. Comparaison avec les informations fournies par la coronarographie. Nouv. Presse Méd., 1982, 111:3491-3494).

Les dosages du cholestérol et des triglycérides, qui font partie depuis longtemps de la panoplie des analyses biologiques, ont révélé leur insuffisance lorsqu'on s'est aperçu que le risque athérogène n'était pas le même selon la classe de lipoprotéines qui transporte ces lipides (Gordon T., Kannel W.B., Castelli W.P., Dawber T.A. Lipoproteins, cardiovascular disease and death. The Framingham study. Arch. Intern. Méd., 1981, 141:1128-1131).

L'utilisation de la classification des lipoprotéines en fonction de critères physicochimiques tels que la charge électrique, la densité, la taille ou l'interaction avec des polyanions a permis de révéler qu'il existait dans le plasma des lipoprotéines athérogènes (LDL) favorisant le dépôt de cholestérol dans les tissus et des lipoprotéines "protectrices" (HDL) captant le cholestérol tissulaire pour le ramener au foie en vue de son excrétion (Polonovski J., Beucler I. Structure et métabolisme des lipoprotéines plasmatiques. Pathol. Biol., 1983, 31:225-234).

La découverte de nombreuses apolipoprotéines, fractions protéiques des lipoprotéines, a conduit au développement de méthodes immunologiques permettant de les quantifier et de classer les lipoprotéines en fonction de leur composition protéique (Fruchart JC., Clavey V., Vanhoutte G. Méthodes d'exploration biochimique des hyperlipoprotéinémies. Pathol. Biol., 1983, 31:235-245). C'est ainsi que les dosages de l'apolipoprotéine A-I, fraction protéique majeure des HDL, et de l'apolipoprotéine B, fraction protéique majeure des LDL sont entrés dans la pratique courante.

Cependant, l'importance de la fraction protéique des lipoprotéines (apolipoprotéines) dans le transport des lipides, les interactions lipoprotéines récepteurs (Brown M.S., Goldstein J.L. Lipoproteins receptors in the liver. J. Clin. Invest., 1983, 72:743-747) et la régulation de l'activité des enzymes impliquées dans le métabolisme des lipoprotéines (Fielding C.J., Shore V.G., Fielding P.E. A protein cofactor of lecithin : cholesterol acyltransferase. Biochem. Biophys. Res. Comm., 1972, 46:1493-1498 ; Jahn C.E., Osborne J.C., Shaefer E.J., Brewer H.B. Activation of the enzyme activity of hepatic lipase by apolipoprotein A-II. Eur. J. Biochem., 1983, 131:25-29 ; Assman G., Mensel H.J. Apolipoprotein disorders. Ric. Clin. Lab., 1982, 12:63-81 : Catapano A.L. Apo C-II and LPL activity. Ric. Clin. Lab., 1982, 12:35-40 ; Polonovski J., Glangeaud D., Freundenthal M.C. La lipoprotéine lipase et son action sur les lipoprotéines de très basse densité. In : Exposés annuels de biochimie médicale, Ed. Masson, 1982, 35: 13-37) a conduit de nombreux auteurs (Fruchart J.C., Puchois P. Méthodes d'analyse des lipoprotéines. Ann. Biol. Clin., 1986, 44:551-555 ; Alaupovic P. The role of apolipoproteins in lipid transport processes. Ric. Clin. Lab., 1982, 12:3-21 ; Atmeh R.F., Shepherd J., Packard C.J. Subpopulations of apolipoproteins A-I in human high density lipoproteins. Their metabolic properties and response to drug therapy. Biochim. Biophys. Acta, 1983, 751:175-188 ; Salmon S., Van Wanbeke A., Theron L., Ayrault-Jarrier M., Polonovski J. Apolipoprotéines associées aux lipoprotéines B dans les lipoprotéines de basse densité du sérum humain. Biochim. Biophys. Acta, 1982, 710:297-305) à utiliser les apolipoprotéines comme marqueurs spécifiques pour la caractérisation des

lipoprotéines. Selon ce concept, les lipoprotéines sont en fait un ensemble de particules composées de lipides associés à une apolipoprotéine (particule simple) ou à plusieurs apolipoprotéines (particule complexe). Les particules ainsi définies pourraient représenter l'unité de base du métabolisme des lipoprotéines et toute dyslipoprotéinémie pourrait être caractérisée par un profil distinct de particules lipoprotéiques.

Un certain nombre d'analyses immunologiques sophistiquées ont donc été développées pour quantifier les apolipoprotéines.

Ces méthodes utilisent l'immunoenzymologie et ne sont applicables que dans des laboratoires de recherche.

Elles ont permis de montrer l'hétérogénéité fonctionnelle des lipoprotéines de haute et de basse densité.

Par exemple, on a pu prouver que les HDL contiennent deux types de particules : celles qui contiennent l'apolipoprotéine AI et l'apolipoprotéine AII (LpAI:AII) et celles qui contiennent l'apolipoprotéine AI mais pas l'apolipoprotéine AII (LpAI).

Des travaux effectués sur culture cellulaire ont montré que seules les LpAI sont capables de capter le cholestérol tissulaire et donc d'être protectrices vis-a-vis de l'athérosclérose.

Une étude rétrospective sur sujets coronarographiés a en outre mis en évidence que les sujets à coronaires lésées présentaient une diminution significative des lipoprotéines LpA-I alors que les lipoprotéines LpA-I:A-II ne sont pas modifiées (Puchois P., Bertrand M., Lablanche J.M., Fruchart J.C. Decrease of plasma apo A-I in coronary artery disease is related to lipoprotein particles which contain apo A-I but not apo A-II. Circulation, 1985, 72:III-199).

Enfin, l'étude de sujets consommant de l'alcool régulièrement, en les séparant en cinq groupes selon leur consommation alcoolique hebdomadaire, a montré que les lipoprotéines LpA-I diminuent proportionnellement à la consommation alcoolique, alors que les lipoprotéines LpA-II:A-I augmentent. L'augmentation du cholestérol HDL constatée classiquement lors de la consommation d'alcool correspondrait donc à une augmentation de la fraction LpA-I:A-II non protectrice (Puchois P., Ghahim N., Demarquilly C., Zylberberg G., Fruchart J.C. Effect of alcohol consumption on lipoprotein particles which contain apo A-I and apo A-II (LpA-I:A-II) and apo A-I but not apo A-II (LpA-I). Circulation, 1986, 74:II-383).

Des travaux comparables ont été réalisés sur les lipoprotéines de basse densité et montré l'athérogénicité des particules contenant uniquement l'apolipoprotéine B (LpB) et de celles contenant les apolipoprotéines B et C-III (LpB:C-III) ou B et E (LpB:E) (Fruchart J.C., Luyeye I., Parra H., Slimane M., Fievet C. Les lipoprotéines athérogènes et leur détection immunologique. Bull. Acad. Natle Méd., 1985, 169: 719-728).

La mise au point de nouvelles méthodes d'analyse moléculaire des particules permet donc d'envisager un diagnostic plus précoce et plus précis du risque d'athérosclérose que l'hétérogénéité des lipoproté'ïnes définies par les critères physicochimiques ne permettrait pas. Parallèlement, ces nouvelles techniques permettent de préciser les mécanismes d'action des médicaments normolipoprotéinémiants au niveau moléculaire (Douste-Blazy P., Fievet C., Fruchart J.C., Slimane M., Drouin P., Puchois P., Bernadet P. Effect of fenofibrate on lipoprotein particles predictive of coronary. Arteriosclerosis, 1986, 6/5:564).

Les méthodes proposées jusqu'alors pour l'analyse moléculaire des particules lipoprotéiques sont les suivantes.

On a ainsi proposé la méthode d'immunoprécipitation séquentielle (Alaupovic P. - Mapping of the lipoprotein particles of very low and low densities characterized by apolipoproteins B ,C-I, C-II, C-III and E as their protein moieties. In : Journées du Gerli, Le Touquet, 19-21 juin 1984). Cette méthode permet de quantifier directement les particules lipoprotéiques, mais est longue et trop complexe pour être appropriée en biologie clinique.

On a également développé une méthode immunoenzymatique à double détermination d'anticorps, qui permet l'analyse quantitative des particules au niveau moléculaire (Fruchart J.C., Puchois P. Méthodes d'analyse des lipoprotéines. Ann. Biol. Clin. , 1986, 44:551-555 ; Puchois P., Bertrand M., Lablanche J.M., Fruchart J.C. - Decrease of plasma Apo A-I in coronary artery disease is related to lipoprotein particles which contain Apo A-I but not Apo A-II. In : 58th Scientific Session of the American Heart Association, Washington, 11-14 novembre 1985. Arteriosclerosis, 1985, 5-5, 513).

Cette technique repose sur le fait que les anticorps spécifiques d'une apolipoprotéine donnée, fixés sur une plaque à microtitration retiennent les particules lipoprotéiques qui contiennent cette apolipoprotéine.

L'analyse quantitative des autres apolipoprotéines présentes sur les particules retenues est alors directement réalisée sur la plaque par addition de leurs anticorps correspondants, marqués par une enzyme.

Cette méthode est rapide et fiable, mais nécessite l'utilisation d'un matériel dont ne disposent pas tous les laboratoires.

On sait également que dans un gel sous forme de plaque, contenant uniformément un anticorps

spécifique d'un antigène déterminé, lorsqu'on fait diffuser l'antigène correspondant (immunodiffusion radiale selon Mancini) ou migrer dans un champ électrique l'antigène correspondant (électroimmunodiffusion selon Laurell) il y a formation d'un précipité visible respectivement sous forme d'un anneau ou d'un pic.

Les surfaces couvertes par les précipités sont proportionnelles à la quantité d'antigène mise en jeu et en comparant les résultats obtenus avec des concentrations connues du même antigène, il est possible d'en déduire la quantité d'antigène à doser.

Ces techniques sont également applicables pour doser deux ou plusieurs antigènes indépendants, c'est-à-dire non liés entre eux, en incorporant à ces gels deux ou plusieurs anticorps spécifiques vis-à-vis de chacun des antigènes (Fruchart JC, Nora I, Cachera C, Clavey V, Duthilleul P, and Moschetto Y. Simultaneous measurement of plasma apolipoproteins A1 and B by electrimmunaoassay. Clin Chem 28/1 (1982) 59-62 et CB Laurell. A Screening test for $\alpha$ 1 antitrypsin deficiency. Scand. J. Clin. Lab. Invest. 29 (1972) 247).

Il est ensuite nécessaire à posteriori d'être en mesure de distinguer les immunoprécipités relatifs à chacun des antigènes. Ce repérage peut être effectué par exemple en utilisant une propriété spécifique d'un antigène ou par marquage d'un anticorps déterminé ; ou bien encore d'après les intensités relatives des lignes de précipitation.

On sait également que si les antigènes ne sont pas indépendants, (c'est-à-dire s'il existe une liaison entre eux) le dosage simultané n'est pas possible.

Un cas intermédiaire entre les antigènes liés et les antigènes indépendants est constitué notamment par celui des apolipoprotéines, qui sont telles qu'une apolipoprotéine déterminée est portée par des particules lipoprotéiques, différentes entre elles par leur charge en autres apolipoprotéines.

Deux particules lipoprotéiques, qui portent toutes les deux une même apolipoprotéine déterminée, sont différentes entre elles lorsque l'une des particules contient au moins en outre une autre apolipoprotéine différente des éventuelles autres apolipoprotéines portées par l'autre particule lipoprotéique.

Ce qui vient d'être dit ci-dessus peut être généralisé pour définir que "n" particules lipoprotéiques portant toutes une même apolipoprotéine déterminée, sont différentes entre elles.

Dans ce cas, les particules lipoprotéiques sont indépendantes, mais les apolipoprotéines ne sont pas indépendantes.

Les techniques de dosage des apolipoprotéines sont celles évoquées ci-dessus, mais à ce jour, on ne connait aucune technique simple et fiable de laboratoire susceptible d'être utilisée en analyse clinique de routine, et ne nécessitant ni traitement préalable du milieu biologique contenant des antigènes liés, notamment des apolipoprotéines à doser, ni matériel sophistiqué.

L'un des aspects de l'invention est de proposer un nouveau procédé de dosage simultané d'un ou plusieurs sous-ensembles d'apolipoprotéines, contenus dans un milieu biologique, ne nécessitant pas de modification préalable du milieu biologique, pour éliminer les particules indésirables.

L'un des aspects de l'invention est de proposer un nouveau procédé de dosage direct d'un ou plusieurs sous-ensembles d'apolipoprotéines, simple à mettre en oeuvre dans le cadre d'analyse clinique de routine.

L'un des autres aspects de l'invention est de proposer un procédé rapide et fiable de dosage d'un ou plusieurs sous-ensembles d'apolipoprotéines.

L'un des aspects de l'invention est de proposer un nouveau procédé de dosage d'un ou plusieurs sous-ensembles d'apolipoprotéines ne nécessitant pas l'utilisation d'un matériel sophistiqué.

L'un des autres aspects de l'invention est de proposer des supports plans tels que plaques ou films prêts à l'emploi pour la mise en oeuvre du dosage direct d'un ou plusieurs sous-ensembles d'apolipoprotéines, contenues dans un milieu biologique, sans traitement préalable dudit milieu biologique.

L'un des autres aspects de l'invention est de proposer un kit ou nécessaire de dosage permettant le dosage direct simple, rapide et fiable d'un ou plusieurs sous-ensembles d'apolipoprotéines.

Ces différents aspects de l'invention reposent sur la constatation tout à fait inattendue que deux groupes de particules (a) et (b), distinctes et indépendantes, (a) portant un antigène I, et (b) portant le même antigène I et un autre antigène II, différent de l'antigène I, lesquelles particules (a) et (b) sont contenues dans un milieu biologique non préalablement traité peuvent se comporter, vis-à-vis de la précipitation, lorsqu'elles sont mises en présence d'un mélange d'anticorps anti I et d'anticorps anti II, dans des conditions appropriées, de la même façon que si de façon séparée, les particules (b) étaient mises en présence d'anticorps anti II et les particules (a) étaient mises en présence d'anticorps anti I.

On constate donc de façon tout à fait inattendue que, dans des conditions appropriées, les particules (b) comportant à la fois l'antigène I et l'antigène II ne sont précipitées que par les anticorps anti II, et que la présence d'anticorps anti I n'entraîne pas la précipitation de certaines particules (b), et ne perturbe pas la précipitation des particules (b) par les anticorps anti II, ce qui permet le dosage respectivement de l'antigène I porté par les particules (a) et de l'antigène II.

Plus précisément, cette invention repose sur la constatation tout à fait inattendue qu'à partir d'un milieu biologique non préalablement traité et contenant deux groupes de particules (a) et (b) distinctes et indépendantes, (a) portant un antigène I et (b) portant le même antigène I et un autre antigène II et en incorporant dans un gel d'immunodiffusion un mélange d'anticorps anti I et d'anticorps anti II, dans des proportions telles que les anticorps anti II soient en excès par rapport aux anticorps anti I, on obtient deux frontières de précipitation, telles que celle qui est en deça de l'autre correspond à la précipitation des particules (b).

Dans son aspect le plus géneral, cette invention propose donc un procédé pour déterminer simultanément le contenu de chacun de deux ou plusieurs groupes de particules distinctes et indépendantes dans un mélange de particules au moyen d'immunodiffusion différentielle desdits groupes de particules sur un gel; lesdits groupes de particules comprenant :

1- un premier groupe de Particules, dont chacune porte un premier antigène et

2- au moins un deuxième groupe de particules, dont chacune porte un second antigène et au moins certains d'entre eux portent ledit premier antigène; ledit deuxième antigène n'étant pas porté par les particules du premier groupe, le procédé comprenant les étapes suivantes :

- traitement du mélange de particules avec un mélange d'anticorps comprenant un premier anticorps dirigé contre le premier antigène et un deuxième anticorps dirigé contre le deuxième antigène; ledit mélange d'anticorps contenant ledit premier anticorps en quantité au moins suffisante pour précipiter le premier groupe dans le mélange de particules; ledit mélange d'anticorps contenant ledit second antigène en quantité au moins suffisante pour précipiter ledit second groupe dans le mélange de particules et en excès de ladite quantité du premier anticorps, ce par quoi les contenus respectifs de chaque premier et deuxième groupe dans ledit mélange de particules peut être déterminé.

Pour la mise en oeuvre de ce procédé, cette invention propose également un kit pour déterminer simultanément le contenu de chacun de deux ou plusieurs groupes de particules distinctes et indépendantes portant des antigènes dans un mélange de particules portant des antigènes, tels que définis ci-dessus ledit kit comprenant une plaque de gel et un mélange contenant des quantités prédéterminées de particules portant les premier et deuxième antigènes.

Le procédé selon l'invention de dosage simultané par immunodiffusion différentielle sur gel en plaque de sous-ensembles d'apolipoprotéines, appartenant respectivement à des groupes de particules lipoprotéiques contenues dans un milieu biologique, ces groupes de particules lipoprotéiques étant tels qu'ils contiennent une "apolipoprotéine commune", et pouvant être définis de la façon suivante :

- l'un des groupes ci-après appelé premier groupe étant constitué par des particules lipoprotéiques portant toutes la susdite "apolipoprotéine commune", cette apolipoprotéine commune portée par toutes les particules lipoprotéiques du premier groupe constituant le premier sous-ensemble,
- le ou les éventuels groupes, ci-après désignés par groupes intermédiaires, étant tels qu'ils sont constitués par des particules lipoprotéiques dont une partie ou toutes, portent l'apolipoprotéine commune définie ci-dessus, lesquelles particules lipoprotéiques portent toutes, en outre, au moins une apolipoprotéine différente des éventuelles autres apolipoprotéines portées par les particules lipoprotéiques du premier groupe, et dans le cas de plusieurs groupes intermédiaires, les particules lipoprotéiques constituant respectivement chaque groupe intermédiaire portant au moins une apolipoprotéine différente de celles portées par les particules lipoprotéiques du ou des autres groupes intermédiaires, cette ou ces apolipoprotéines portées par les particules lipoprotéiques de chacun des groupes intermédiaires et différentes de l'apolipoprotéine constituant le premier sous ensemble, différentes des éventuelles autres apolipoprotéines portées par les particules lipoprotéiques du premier groupe et différente des éventuelles autres apolipoprotéines portées par les particules lipoprotéiques des autres groupes intermédiaires constituant le ou les sous-ensembles intermédiaires, le dernier groupe étant tel que :
- soit il est constitué par des particules lipoprotéiques contenant toutes "l'apolipoprotéine commune" définie ci-dessus et contenant, en outre, au moins une apolipoprotéine différente des éventuelles autres apolipoprotéines portées par les particules lipoprotéiques du premier groupe et différentes des éventuelles apolipoprotéines portées par les particules lipoprotéiques des éventuels groupes intermédiaires, cette ou ces apolipoprotéine(s) différente(s) de l'apolipoprotéine commune et différente(s) des éventuelles autres apolipoprotéines portées par les particules lipoprotéiques du premier groupe et différentes des éventuelles apolipoprotéines portées par les particules lipoprotéiques des éventuels groupes intermédiaires, constituant le dernier sous-ensemble ;
- soit il est constitué par des particules lipoprotéiques dont, d'une part, une partie porte la susdite "apolipoprotéine commune" définie ci-dessus et porte en outre au moins une apolipoprotéine différente des éventuelles autres apolipoprotéines portées par les particules lipoprotéiques du premier

groupe et différentes des éventuelles apolipoprotéines portées par les particules lipoprotéiques des éventuels groupes intermédiaires, et dont, d'autre part, le reste des particules lipoprotéiques ne porte pas la susdite apolipoprotéine commune définie ci-dessus, mais porte l'une au moins des apolipoprotéines portées par la partie des particules lipoprotéiques susdéfinie, et différente des éventuelles autres apolipoprotéines portées par le premier groupe de particules lipoprotéiques et différentes des éventuelles apolipoprotéines portées par les particules lipoprotéiques des éventuels groupes intermédiaires, cette ou ces apolipoprotéines différentes des éventuelles autres apolipoprotéines portées par les particules lipoprotéiques du premier groupe et différentes des éventuelles apolipoprotéines portées par les particules lipoprotéiques des éventuels groupes intermédiaires constituant le dernier sous-ensemble ;

est caractérisé en ce que :

- on met le milieu biologique contenant les sous-ensembles d'apolipoprotéines à doser en présence d'un mélange d'anticorps contenant
  . d'une part des anticorps dirigés contre la ou chacune des apolipoprotéines constituant le dernier sous-ensemble en quantité suffisante pour précipiter les particules lipoprotéiques du dernier groupe (portant les apolipoprotéines constituant le dernier sous-ensemble) ;
  . d'autre part, éventuellement, des anticorps dirigés contre la ou les apolipoprotéines constituant le ou les sous ensembles intermédiaires en quantité suffisante pour précipiter les particules lipoprotéiques du ou des éventuels groupes intermédiaires (portant les apolipoprotéines constituant les sous-ensembles intermédiaires) ;
  . d'autre part des anticorps dirigés contre l'apolipoprotéine commune sus-définie, constituant le premier sous-ensemble, en quantité suffisante pour précipiter les particules lipoprotéiques du premier groupe (portant l'apolipoprotéine constituant le premier sous-ensemble) ;
- la quantité des susdits anticorps nécessaires à la précipitation des particules lipoprotéiques du dernier groupe (portant les apolipoprotéines constituant le dernier sous-ensemble), étant en excès par rapport à la quantité des éventuels susdits anticorps nécessaires à la précipitation des particules lipoprotéiques du ou des éventuels groupes intermédiaires (portant les apolipoprotéines constituant le ou les éventuels sous-ensembles intermédiaires) ;
- la ou les quantités d'anticorps nécessaires à la précipitation des particules lipoprotéiques des éventuels groupes intermédiaires étant en excès par rapport à la quantité des susdits anticorps nécessaires à la précipitation des particules lipoprotéiques du premier groupe (portant l'apolipoprotéine constituant le premier sous ensemble), et dans le cas de plusieurs groupes intermédiaires, les quantités d'anticorps nécessaires à la précipitation des groupes intermédiaires, étant respectivement en excès les uns par rapport aux autres selon un ordre prédéterminé ; dans un rapport tel que, après immunodiffusion, on obtienne au moins deux frontières distinctes, la frontière d'immunodiffusion la plus proche du point de dépôt du milieu contenant les sous-ensembles à doser correspondant à la précipitation des particules lipoprotéiques portant les apolipoprotéines constituant le dernier sous-ensemble et la frontière d'immunodiffusion la plus éloignée du susdit point de dépôt correspondant à la précipitation des particules lipoprotéiques portant l'apolipoprotéine constituant le premier sous-ensemble, et la ou les frontières éventuelles d'immunodiffusion à une distance intermédiaire entre la frontière la plus proche et la frontière la plus éloignée sus mentionnées, correspondant à la précipitation des éventuelles particules lipoprotéiques portant la ou les apolipoprotéines constituant les sous-ensembles intermédiaires ;
- on mesure les distances maximales respectives entre le point de dépôt et chacune des frontières d'immunodiffusion et on compare chacune des distances par rapport à celles obtenues avec un milieu biologique étalon, contenant des quantités connues d'apolipoprotéines respectives des sous-ensembles.

On rappelle ci-après qu'une particule lipoprotéique précipite sous l'effet d'une réaction antigène-anticorps intervenant entre l'une des apolipoprotéines portées par la particule lipoprotéique et l'anticorps dirigé contre la susdite apolipoprotéine, et que le dosage concerne l'apolipoprotéine portée par la susdite particule lipoprotéique.

On a constaté de façon inattendue que les frontières d'immunodiffusion, (correspondant respectivement à la précipitation du premier groupe de particules lipoprotéiques, du ou des éventuels groupes intermédiaires de particules lipoprotéiques et du dernier groupe de particules lipoprotéiques auxquelles appartiennent respectivement le premier sous-ensemble d'apolipoprotéines à doser, le ou les éventuels sous-ensembles intermédiaires d'apolipoprotéines à doser et le dernier sous-ensemble d'apolipoprotéines à doser), lorsque le milieu biologique est mis sans traitement préalable en présence d'un mélange d'anticorps définis ci-dessus, sont identiques aux frontières d'immunodiffusion obtenues dans des conditions, ou après avoir

séparé les différents groupes de particules lipoprotéiques définies ci-dessus,
on met

- d'une part le premier groupe de particules lipoprotéiques en contact avec des anticorps dirigés contre l'apolipoprotéine commune constituant le premier sous-ensemble défini ci-dessus, en quantité identique à celle utilisée ci-dessus pour précipiter les particules lipoprotéiques du premier groupe non séparées du milieu biologique et portant l'apolipoprotéine constituant le premier sous-ensemble,
  on met
- d'autre part le ou les éventuels groupes intermédiaires de particules lipoprotéiques en contact avec des anticorps dirigés contre les apolipoprotéines constituant le ou les éventuels sous-ensembles intermédiaires définis ci-dessus, en quantité identique à celle utilisée ci-dessus pour précipiter les particules lipoprotéiques du ou des éventuels groupes intermédiaires non séparées du milieu biologique et portant les éventuelles apolipoprotéines constituant le ou les éventuels sous-ensembles intermédiaires,
  on met
- d'autre part le dernier groupe de particules lipoprotéiques en contact avec des anticorps dirigés contre les apolipoprotéines constituant le dernier sous-ensemble défini ci-dessus en quantité identique à celle utilisée ci-dessus pour précipiter les particules lipoprotéiques du dernier groupe, non séparées du milieu biologique et portant les apolipoprotéines constituant le dernier sous-ensemble.

En ce qui concerne les éventuelles frontières d'immunodiffusion intermédiaires, elles sont positionnées les unes par rapport aux autres dans l'ordre prédéterminé des excès d'anticorps mentionnés ci-dessus, la frontière d'immunodiffusion intermédiaire la plus proche de la frontière d'immunodiffusion correspondant à la précipitation du dernier groupe étant celle qui correspond à la précipitation d'un groupe intermédiaire par les anticorps correspondants, dont la quantité est en excès par rapport à toutes les autres quantités d'anticorps nécessaires à la précipitation des autres groupes intermédiaires.

En d'autres termes, un pic correspondant à la précipitation d'un groupe intermédiaire de particules lipoprotéiques se trouve en deça d'un autre pic, correspondant à la précipitation d'un autre groupe intermédiaire de particules lipoprotéiques, lorsque la quanité d'anticorps entraînent à la précipitation du premier groupe intermédiaire dont question ci-dessus est en excès par rapport à la quantité d'anticorps entraînent la précipitation de l'autre groupe intermédiaire.

Le procédé de dosage simultané par immunodiffusion différentielle sur gel en plaque d'au moins trois sous-ensembles d'apolipoprotéines, appartenant respectivement à des groupes de particules lipoprotéiques contenues dans un milieu biologique, ces trois groupes de particules lipoprotéiques étant tels qu'ils contiennent une "apolipoprotéine commune", et pouvant être définis de la façon suivante :

- l'un des groupes ci-après appelé premier groupe étant constitué par des particules lipoprotéiques portant toutes la susdite "apolipoprotéine commune", et portant d'éventuelles autres apolipoprotéines $X_m$, cette "apolipoprotéine commune" portée par toutes les particules lipoprotéiques du premier groupe constituant le premier sous-ensemble,
- un autre groupe, ci-après désigné par deuxième groupe ou groupe intermédiaire, étant tel que :
  - soit il est constitué par des particules lipoprotéiques portant toutes "l'apolipoprotéine commune" définie ci-dessus, et portant en outre au moins une apolipoprotéine $Y_n$ différente des éventuelles autres apolipoprotéines $X_m$ portées par les particules lipoprotéiques du premier groupe, cette ou ces apolipoprotéine(s) $Y_n$ constituant le deuxième sous-ensemble ou le sous-ensemble intermédiaire,
  - soit il est constitué par des particules lipoprotéiques dont d'une part une partie porte la susdite "apolipoprotéine commune", constituant le premier sous-ensemble, et porte, en outre, au moins une apolipoprotéine $Z_p$, différente des éventuelles autres apolipoprotéines $X_m$ portées par les particules lipoprotéiques du premier groupe, et dont, d'autre part, le reste des particules lipoprotéiques ne porte pas la susdite "apolipoprotéine commune", mais porte au moins une des apolipoprotéines $Z_p$ portées par la partie des particules lipoprotéiques sus-définie et différentes des éventuelles autres apolipoprotéines $X_m$ portées par le premier groupe de particules lipoprotéiques, cette ou ces apolipoprotéine(s) $Z_p$ constituant le deuxième sous-ensemble, ou le sous-ensemble intermédiaire,
- le troisième groupe, ou dernier groupe, étant tel que
  - soit il est constitué par des particules lipoprotéiques contenant toutes "l'apolipoprotéine commune" définie ci-dessus et contenant en outre au moins une apolipoprotéine $W_q$, différente d'une part des éventuelles autres apolipoprotéines $X_m$ portées par les particules lipoprotéiques du premier groupe, et d'autre part des apolipoprotéines portées par les particules lipoprotéiques du deuxième groupe, cette ou ces apolipoprotéine(s) $W_q$ constituant le troisième sous-ensemble, ou dernier sous

ensemble ;

- soit il est constitué par des particules lipoprotéiques dont, d'une part, une partie porte la susdite "apolipoprotéine commune" et porte en outre au moins une apolipoprotéine $T_1$ différente à la fois des éventuelles autres apolipoprotéines $X_m$ portées par les particules lipoprotéiques du premier groupe, et des apolipoprotéines portées par les particules lipoprotéiques du deuxième groupe, et dont, d'autre part, le reste des particules lipoprotéiques ne porte pas la susdite "apolipoprotéine commune", mais porte l'une au moins des apolipoprotéines $T_1$ portées par la partie des particules lipoprotéiques susdéfinie, cette ou ces apolipoprotéines $T_1$ constituant le troisième sous-ensemble, ou dernier sous-ensemble ;

est caractérisé en ce que :

- on met le milieu biologique contenant les sous-ensembles à doser en présence d'un mélange d'anticorps contenant
  . des anticorps dirigés contre la ou chacune des apolipoprotéines constituant le troisième ou dernier sous-ensemble en quantité suffisante pour précipiter les particules lipoprotéiques du troisième ou dernier groupe
  . des anticorps dirigés contre la ou chacune des apolipoprotéines constituant le deuxième sous-ensemble, ou sous-ensemble intermédiaire, en quantité suffisante pour précipiter les particules lipoprotéiques du deuxième groupe, ou groupe intermédiaire ;
  . des anticorps dirigés contre "l'apolipoprotéine commune" aux sous-ensembles, et constituant le premier sous-ensemble, en quantité suffisante pour précipiter les particules lipoprotéiques du premier groupe ;
- la quantité des susdits anticorps nécessaires à la précipitation des particules lipoprotéiques du troisième ou dernier groupe étant en excès par rapport à la quantité des susdits anticorps nécessaires à la précipitation des particules lipoprotéiques du deuxième groupe ou groupe intermédiaire, cette dernière quantité des susdits anticorps nécessaires à la précipitation des particules lipoprotéiques du deuxième groupe ou groupe intermédiaire étant elle-même en excès par rapport à la quantité des susdits anticorps nécessaires à la précipitation des particules lipoprotéiques portant "l'apolipoprotéine commune" constituant le premier sous-ensemble, dans un rapport tel que, après immunodiffusion, on obtienne trois frontières distinctes, la frontière d'immunodiffusion la plus proche du point de dépôt du milieu contenant les sous-ensembles à doser, correspondant à la précipitation des particules lipoprotéiques du troisième ou dernier groupe, la frontière d'immunodiffusion la plus éloignée du susdit point de dépôt correspondant à la précipitation des particules lipoprotéiques du premier groupe, la frontière d'immunodiffusion intermédiaire entre la frontière la plus proche et la frontière la plus éloignée susmentionnées correspondant à la précipitation des particules lipoprotéiques du deuxième groupe ;
- on mesure les distances maximales respectives entre le point de dépôt et chacune des trois frontières d'immunodiffusion et on compare chacune des distances par rapport à celles obtenues avec un milieu étalon, contenant des quantités connues d'apolipoprotéines respectives des trois sous-ensembles.

Selon un mode de réalisation préféré du procédé de l'invention, le groupe sus-désigné par deuxième groupe ou groupe intermédiaire est de préférence constitué par des particules lipoprotéiques portant toutes "l'apolipoprotéine commune" définie ci-dessus, et portant en outre au moins une apolipoprotéine $Y_n$ différente des éventuelles autres apolipoprotéines $X_m$ portées par les particules lipoprotéiques du premier groupe, cette ou ces apolipoprotéine(s) $Y_n$ constituant le deuxième sous-ensemble ou le sous-ensemble intermédiaire.

Le procédé selon l'invention de dosage simultané par immunodiffusion différentielle sur gel en plaque d'au moins deux sous-ensembles d'apolipoprotéines, appartenant respectivement à au moins deux groupes de particules lipoprotéiques contenues dans un milieu biologique, ces deux groupes de particules lipoprotéiques étant tels qu'ils contiennent une "apolipoprotéine commune",

- l'un des groupes ci-après appelé premier groupe étant constitué par des particules lipoprotéiques portant toutes la susdite "apolipoprotéine commune", et portant d'éventuelles autres apolipoprotéines $X_m$, cette apolipoprotéine commune portée par toutes les particules lipoprotéiques du premier groupe constituant le premier sous-ensemble,
- l'autre groupe, ci-après désigné par deuxième groupe étant tel que :
  - soit il est constitué par des particules lipoprotéiques contenant toutes "l'apolipoprotéine commune" définie ci-dessus et portant en outre au moins une apolipoprotéine $Y_n$ différente des éventuelles autres apolipoprotéines $X_m$ portées par les particules lipoprotéiques du premier groupe, cette ou ces apolipoprotéine(s) $Y_n$ constituant le deuxième sous-ensemble ;
- soit il est constitué par des particules lipoprotéiques dont, d'une part, une partie porte la susdite

"apolipoprotéine commune" constituant le premier sous-ensemble et porte, en outre, au moins une apolipoprotéine $Z_p$ différente des éventuelles autres apolipoprotéines $X_m$ portées par les particules lipo- protéiques du premier groupe et dont, d'autre part, le reste des particules lipoprotéiques ne porte pas la susdite "apolipoprotéine commune" constituant le premier sous-ensemble mais porte l'une au moins des apolipoprotéines $Z_p$ portées par la partie des par- ticules lipoprotéiques susdéfinie et différente des éventuelles autres apolipoprotéines $X_m$ portées par le premier groupe de particules lipoprotéiques, cette ou ces apolipoprotéines $Z_p$ constituant le deuxième sous-ensemble ;
est caractérisé en ce que :

- on met le milieu biologique contenant les deux sous-ensembles d'apolipoprotéine à doser en présence d'un mélange d'anticorps contenant
  . d'une part des anticorps dirigés contre la ou chacune des apolipoprotéines constituant le deuxième sous-ensemble ($Y_n$ ou $Z_p$) en quantité suffisante pour précipiter les particules lipoprotéiques du deuxième groupe ;
  . d'autre part des anticorps dirigés contre la susdite "apolipoprotéine commune", constituant le premier sous-ensemble, en quantité suffisante pour précipiter les particules lipoprotéiques du premier groupe ;
- la quantité des susdits anticorps nécessaires à la précipitation des particules lipoprotéiques du deuxième groupe étant en excès par rapport à la quantité des susdits anticorps nécessaires à la précipitation des particules lipoprotéiques du premier groupe, dans un rapport tel que, après immunodiffusion, on obtienne deux frontières distinctes, la frontière d'immunodiffusion la plus proche du point de dépôt du milieu contenant les deux sous-ensembles à doser correspondant à la précipitation des particules lipoprotéiques du deuxième groupe et la frontière d'immunodiffusion la plus éloignée du susdit point de dépôt correspondant à la précipitation des particules lipoprotéiques du premier groupe, ces deux frontières étant respectivement identiques à celles obtenues dans les conditions où, après avoir séparé les deux groupes de particules lipoprotéiques contenant respectivement le premier et le deuxième sous-ensembles d'apolipoprotéines, on met, d'une part, le premier groupe de particules lipoprotéiques en contact avec des anticorps dirigés contre l'apolipoprotéine commune constituant le premier sous-ensemble défini ci-dessus, en quantité identique à celle utilisée ci-dessus pour précipiter les particules lipoprotéiques du premier groupe, non séparées du milieu biologique et portant l'apolipoprotéine constituant le premier sous-ensemble, et on met, d'autre part, le deuxième groupe de particules lipoprotéiques en contact avec des anticorps dirigés contre les apolipoprotéines constituant le deuxième sous-ensemble défini ci-dessus, en quantité identique à celle utilisée ci-dessus pour précipiter les particules lipoprotéiques du deuxième groupe, non séparées du milieu biologique et portant les apolipoprotéines constituant le deuxième sous-ensemble ;
  - on mesure les distances maximales respectives entre le point de dépôt et chacune des deux frontières d'immunodiffusion et on compare chacune des distances par rapport à celles obtenues avec un milieu biologique étalon, contenant des quantités connues d'apolipoprotéines respectives des deux sous-ensembles.

On a constaté de façon inattendue que les frontières d'immunodiffusion correspondent à la précipitation respective de chacun des groupes de particules lipoprotéiques auxquelles appartiennent, comme indiqué ci-dessus, respectivement chacun des sous-ensembles d'apolipoprotéine à doser.

On donne ci-après, à titre d'exemple, la constitution de trois sous-ensembles d'apolipoprotéines à doser, appartenant à trois groupes de particules lipoprotéiques.

Le premier sous-ensemble est constitué par une apolipoprotéine, portée par toutes les particules lipoprotéiques du premier groupe et qui est également portée par une partie au moins des particules lipoprotéiques du deuxième et du troisième groupe, ou qui est portée par toutes les particules lipoprotéiques du deuxième groupe et par une partie au moins des particules lipoprotéiques du troisième groupe.

Le deuxième sous-ensemble est constitué par une apolipoprotéine différente de l'apolipoprotéine qui constitue le premier sous-ensemble, mais portée par une partie au moins des particules lipoprotéiques du troisième groupe.

Le troisième sous-ensemble est constitué par des apolipoprotéines différentes des apolipoprotéines constituant respectivement le premier et le deuxième sous-ensemble et différente des éventuelles autres apolipoprotéines portées sur les particules lipoprotéiques du premier et du deuxième groupe.

Le procédé selon l'invention de dosage simultané par immunodiffusion différentielle sur gel en plaque de deux sous-ensembles d'apolipoprotéine, appartenant respectivement à deux groupes de particules lipoprotéiques contenues dans un milieu biologique, ces deux groupes de particules lipoprotéiques étant tels qu'ils contiennent une "apolipoprotéine commune",

- l'un des groupes ci-après appelé premier groupe étant constitué par des particules lipoprotéiques

portant toutes la susdite "apolipoprotéine commune", et portant d'éventuelles autres apolipoprotéines $X_m$, cette apolipoprotéine commune portée par toutes les particules lipoprotéiques du premier groupe constituant le premier sous-ensemble, et l'autre groupe
ci-après désigné par deuxième groupe étant tel que :

- soit il est constitué par des particules lipoprotéiques contenant toutes "l'apolipoprotéine commune" définie ci-dessus et portant en outre au moins une apolipoprotéine $Y_n$ différente des éventuelles autres apolipoprotéines $X_m$ portées par les particules lipoprotéiques du premier groupe, cette ou ces apolipoprotéine(s) $Y_n$ constituant le deuxième sous-ensemble ;
- soit il est constitué par des particules lipoprotéiques dont, d'une part, une partie porte la susdite "apolipoprotéine commune" du premier groupe et porte en outre au moins une apolipoprotéine $Z_p$ différente des éventuelles autres apolipoprotéines $X_m$ portées par les particules lipoprotéiques du premier groupe et dont, d'autre part, le reste des particules lipoprotéiquéiques ne porte pas la susdite "apolipoprotéine commune" constituant le premier sous-ensemble, mais porte l'une au moins des apolipoprotéines Zp portées par la partie des particules lipoprotéiques susdéfinie et différente des éventuelles autres apolipoprotéines $X_m$ portées par le premier groupe de particules lipoprotéiques, cette ou ces apolipoprotéines Zp constituant le deuxième sous-ensemble ;

est caractérisé en ce que :

- on met le milieu biologique contenant les deux sous-ensembles d'apolipoprotéine à doser en présence d'un mélange d'anticorps contenant
  . d'une part des anticorps dirigés contre la ou chacune des apolipoprotéines du deuxième sous-ensemble ($Y_n$ ou $Z_p$) en quantité suffisante pour précipiter les particules lipoprotéiques du deuxième groupe ;
  . d'autre part des anticorps dirigés contre "l'apolipoprotéine commune" aux deux sous-ensembles en quantité suffisante pour précipiter les particules lipoprotéiques du premier groupe ;
- la quantité des susdits anticorps nécessaires à la précipitation des particules lipoprotéiques du deuxième groupe étant en excès par rapport à la quantité des susdits anticorps nécessaires à la précipitation des particules lipoprotéiques du premier groupe, dans un rapport tel que, après immunodiffusion, on obtienne deux frontières distinctes, la frontière d'immunodiffusion la plus proche du point de dépôt du milieu contenant les deux sous-ensembles à doser correspondant à la précipitation des particules lipoprotéiques du deuxième groupe et la frontière d'immunodiffusion la plus éloignée du susdit point de dépôt correspondant à la précipitation des particules lipoprotéiques du premier groupe, ces deux frontières étant respectivement identiques à celles obtenues dans les conditions où, après avoir séparé les deux groupes de particules lipoprotéiques contenant respectivement le premier et le deuxième sous-ensembles d'apolipoprotéines, on met, d'une part, le premier groupe de particules lipoprotéiques en contact avec des anticorps dirigés contre l'apolipoprotéine commune du premier groupe en quantité identique à celle utilisée ci-dessus pour précipiter les particules lipoprotéiques et non séparées du milieu biologique, portant l'apolipoprotéine du premier sous-ensemble, et on met, d'autre part, le deuxième groupe de particules lipoprotéiques en contact avec des anticorps dirigés contre la ou les apolipoprotérotéines du deuxième sous-ensemble, différente(s) de l'apolipoprotéine du premier sous-ensemble, et différente des éventuelles autre apolipoprotéines portées par les particules lipoprotéiques du premier groupe en quantité identique à celle utilisée ci-dessus pour précipiter les particules lipoprotéiques et non séparées du milieu biologique, portant les apolipoprotéines du deuxième sous-ensemble ;
  - on mesure les distances maximales respectives entre le point de dépôt et chacune des deux frontières d'immunodiffusion et on compare chacune des distances par rapport à celles obtenues avec un milieu biologique étalon, contenant des quantités connues d'apolipoprotéines respectives des deux sous-ensembles.

Le sous-ensemble d'apolipoprotéine qui a été désigné ci-dessus, et qui sera désigné dans la suite par premier sous-ensemble, est constitué d'une seule apolipoprotéine, qui est en fait l'apolipoprotéine commune à toutes les particules lipoprotéiques du groupe de particules désigné, dans ce qui précède et ce qui suit, par premier groupe de particules lipoprotéiques. Ceci n'exclut pas que les particules lipoprotéiques du premier groupe contiennent également d'autres apolipoprotéines.

Le sous-ensemble d'apolipoprotéines qui a été désigné, dans ce qui précède et ce qui suit, par deuxième sous-ensemble, est constitué d'une ou plusieurs apolipoprotéines, différentes d'une part de l'apolipoprotéine commune définie ci-dessus et différentes d'autre part des éventuelles autres apolipoprotéines portées par les particules lipoprotéiques du premier groupe.

Cependant, la ou les apolipoprotéines du deuxième sous-ensemble sont portées par des particules lipoprotéiques, dont une partie au moins porte la susdite apolipoprotéine commune.

Cependant, la susdite apolipoprotéine commune, qui est portée dans une partie au moins des particules lipoprotéiques du deuxième groupe, ne fait pas partie du deuxième sous-ensemble d'apolipoprotéine.

Le deuxième groupe de particules lipoprotéiques peut donc avantageusement être constitué

- soit par des particules lipoprotéiques contenant toutes l'apolipoprotéine commune, et au moins une apolipoprotéine différente de l'apolipoprotéine commune et différente des éventuelles autres apolipoprotéines portées par les particules lipoprotéiques du premier groupe,
- soit par des particules dont une partie contient la susdite apolipoprotéine commune et, en outre, au moins une apolipoprotéine différente de cette apolipoprotéine commune, et différente des éventuelles autres apolipoprotéines portées par les particules lipoprotéiques du premier groupe et dont les reste des particules lipoprotéiques ne contient pas la susdite apolipoprotéine commune, mais contient au moins une apolipoprotéine identique à celle définie dans la susdite partie comme étant différente de l'apolipoprotéine commune et différente des éventuelles autres apolipoprotéines portées par les particules lipoprotéiques du premier groupe.

Selon un mode de réalisation préféré du procédé selon l'invention, le premier sous-ensemble est constitué d'une seule apolipoprotéine portée par des particules lipoprotéiques libres et identiques entre elles, constituant le premier groupe.

On définit par particule lipoprotéique libre, une particule ne contenant qu'une seule apolipoprotéine.

Selon un autre mode de réalisation du procédé de l'invention, le premier groupe est constitué d'une part par des particules lipoprotéiques libres et identiques entre elles, portant l'apolipoprotéine commune constituant le premier sous-ensemble, et d'autre part par des particules lipoprotéiques complexes portant, outre la susdite apolipoprotéine commune, au moins une apolipoprotéine différente de la susdite apolipoprotéine commune et différente des apolipoprotéines constituant le deuxième sous-ensemble.

Par particules lipoprotéiques complexes, on désigne une particule portant au moins deux apolipoprotéines.

Selon un mode de réalisation préféré du procédé de l'invention, le deuxième sous-ensemble est constitué par une apolipoprotéine différente de l'apolipoprotéine commune constituant le premier sous ensemble et différente des éventuelles autres apolipoprotéines portées par les particules lipoprotéiques du premier groupe, laquelle apolipoprotéine constituant le deuxième sous-ensemble est portée par des particules lipoprotéiques complexes, portant en outre toutes la susdite apolipoprotéine constituant le premier sous-ensemble.

Selon un autre mode de réalisation préféré du procédé de l'invention, le deuxième sous-ensemble est constitué par au moins une apolipoprotéine différente de l'apolipoprotéine constituant le premier sous-ensemble et différente des éventuelles autres apolipoprotéines portées par les particules lipoprotéiques du premier groupe, laquelle apolipoprotéine du deuxième sous-ensemble est portée d'une part par des particules lipoprotéiques complexes, portant en outre l'apolipoprotéine constituant le premier sous-ensemble, et est portée d'autre part par des particules lipoprotéiques simples ou complexes, ne portant pas l'apolipoprotéine constituant le premier sous-ensemble.

En ce qui concerne le milieu biologique, il s'agit notamment du sang.

Comme anticorps, on peut utiliser des anticorps monoclonaux, des anticorps polyclonaux ou bien encore des anticorps oligoclonaux, c'est-à-dire des anticorps obtenus par immunisation avec des peptides de synthèse copiant la structure des apolipoprotéines.

En ce qui concerne l'immunodiffusion, on peut avoir recours à la méthode d'électroimmunodiffusion de Laureil, comme par exemple décrite dans Clin. Chem. 1974, 20(6), 676-681.

Les frontières d'immunodiffusion obtenues sont alors des contours de pics. Etant donné que les surfaces couvertes par les particules lipoprotéiques précipitées sont proportionnelles aux quantités d'apolipoprotéines à doser, et que les pics ont sensiblement tous la même base, les quantité d'apolipoprotéines à déterminer par rapport à des pics obtenus à partir d'un milieu biologique étalon, sont déduites d'après la hauteur des pics.

Cette méthode est particulièrement avantageuse car elle est rapide.

En ce qui concerne l'immunodiffusion, on peut avoir recours à la méthode d'immunodiffusion radiale selon Mancini, telle que par exemple décrite dans Mancini G. Carbonara AO, et Heremans JF. Immunochemical quantitation of antigens by single radial immunodiffusion. Immunochemistry 1965, 2, 235.

Les frontières d'immunodiffusion sont des cercles, correspondant à des contours d'anneaux.

Etant donné que les surfaces couvertes par les particules lipoprotéiques précipitées sont proportionnelles aux quantités d'apolipoprotéines à doser, les quantités d'apolipoprotéines à déterminer par rapport à des anneaux obtenus à partir d'un milieu biologique étalon, sont déduites d'après le carré du diamètre des cercles correspondants.

Selon un mode de réalisation préféré du procédé de l'invention, la quantité minimale à utiliser

d'anticorps dirigés contre la ou les apolipoprotéines du deuxième sous-ensemble est déterminée dès l'apparition de deux frontières distinctes d'immunodiffusion.

Dans le cadre de l'invention, la quantité d'anticorps dirigés contre la ou les apolipoprotéines du deuxième sous-ensemble nécessaire à faire précipiter les particules lipoprotéiques du deuxième groupe, est ajoutée en excès par rapport à la quantité d'anticorps dirigés contre l'apolipoprotéine du premier sous-ensemble, nécessaire à faire précipiter les particules lipoprotéiques du premier groupe, ce qui donne une hauteur de pic inférieure à la hauteur du pic résultant de la précipitation du premier groupe.

La quantité d'anticorps dirigés contre la ou les apolipoprotéines du deuxième sous-ensemble à ajouter pour faire précipiter les particules lipoprotéiques du deuxième groupe, n'est pas limitée en valeur maximale.

Cependant, au fur et à mesure que l'on augmente la quantité d'anticorps dirigés contre la ou les apolipoprotéines du deuxième sous-ensemble, la hauteur du pic correspondant à la précipitation des particules lipoprotéiques du deuxième groupe diminue, et il existe une quantité d'anticorps dirigés contre les apolipoprotéines du deuxième sous-ensemble, à partir de laquelle les particules lipoprotéiques du deuxième groupe sont précipitées sur le point de dépôt du milieu biologique.

Au delà de cette quantité, il n'y a plus diffusion des particules lipoprotéiques du deuxième groupe. Dans ce cas, seule l'apolipoprotéine du premier sous ensemble est dosée.

Selon un mode de réalisation avantageux du procédé de l'invention, la quantité d'anticorps dirigés contre la, ou les apolipoprotéines du deuxième sous ensemble, est située dans une gamme dont la valeur minimale est déterminée dès l'apparition de deux frontières distinctes d'immunodiffusion et dont la valeur maximale est déterminée dès que les particules lipoprotéiques portant les apolipoprotéines du deuxième sous-ensemble ne diffusent plus et sont précipitées sur le point de dépôt du milieu biologique, la frontière d'immunodiffusion correspondant à la précipitation des particules lipoprotéiques du premier groupe n'étant pas modifiée lorsque la quantité d'anticorps dirigés contre la ou les apolipoprotéines du deuxième sous-ensemble est supérieure à la valeur minimum à partir de laquelle on observe l'apparition de deux frontières distinctes d'immunodiffusion.

Selon un autre mode de réalisation préféré du procédé de l'invention, la quantité d'anticorps dirigés contre la ou les apolipoprotéines du deuxième sous-ensemble est telle que la distance maximale entre le point de dépôt et la frontière d'immunodiffusion correspondant la précipitation des particules lipoprotéiques contenant les apolipoprotéines du deuxième sous-ensemble est d'environ 1/2 à environ 1/8, avantageusement d'environ 1/3 à environ 1/4, de la distance maximale entre le point de dépôt et la frontière d'immunodiffusion correspondant à la précipitation des particules lipoprotéiques contenant l'apolipoprotéine du premier sous-ensemble.

Selon un autre mode de réalisation préféré du procédé de l'invention, l'étalonnage est effectué à partir d'un milieu dans lequel le premier sous-ensemble contient une quantité connue (A) d'apolipoprotéine, laquelle est également portée par une partie au moins des particules lipoprotéiques du deuxième groupe, le deuxième sous-ensemble contient une ou des quantités connues $(B)_i$ d'apolipoprotéine(s) différente(s) de l'apolipoprotéine du premier cous ensemble et différente des éventuelles autres apolipoprotéines portées par les particules lipoprotéiques du premier groupe :

- en mettant sur la plaque des anticorps dirigés respectivement contre chacune des apolipoprotéines du deuxième sous-ensemble en quantité nécessaire pour précipiter les particules lipoprotéiques du deuxième groupe, chacune de ces quantités étant respectivement en excès par rapport aux anticorps nécessaires à précipiter les particules lipoprotéiques du premier groupe, l'excès en anticorps dirigés contre chacune des apolipoprotéines du deuxième sous-ensemble par rapport aux anticorps dirigés contre l'apolipoprotéine du premier sous-ensemble pouvant être exprimé par la relation suivante :

$$V_{2i} T_{2i} \times \frac{B_i}{A} \quad \text{supérieur à } V_1 T_1 ,$$

  dans laquelle $V_1$, $T_1$ représentent respectivement le volume et le titre des anticorps dirigés contre l'apolipoprotéine du premier sous-ensemble et $V_{2i}$, $T_{2i}$ représentent respectivement les volumes et les titres des anticorps dirigés respectivement contre chacune des apolipoprotéines du deuxième sous-ensemble.

Lorsqu'on utilise la technique d'électroimmunodiffusion, étant donné que l'erreur relative de mesure de la hauteur d'un pic est d'autant plus faible que la hauteur du pic est plus élevée, il convient de profiter au mieux des dimensions utiles de la plaque pour les pics.

Pour ce faire, on détermine :

1°) la quantité d'anticorps $V_1$ , (destinés à précipiter les particules lipoprotéiques portant l'apolipoprotéine du premier sous-ensemble, laquelle apolipoprotéine est également portée par une partie au moins des particules lipoprotéiques du deuxième groupe), pour que la hauteur du pic obtenue soit $H_1$, cette détermination ayant lieu en mettant sur la plaque une quantité connue d'anticorps $v_1$ correspondant à la précipitation des particules lipoprotéiques du premier groupe, ce qui donne une hauteur $h_1$, la quantité $V_1$ étant alors calculée selon la formule suivante :

$$V_1 = x\,\% \;\frac{h_1 v_1}{H_1}$$

x% représentant le pourcentage de l'apolipoprotéine constituant le premier sous-ensemble, par rapport à la quantité totale de cette apolipoprotéine contenue dans le milieu,

et on détermine

2°) la quantité d'anticorps $V_2$ (destinés à précipiter les particules lipoprotéiques portant la ou les apolipoprotéines du deuxième sous-ensemble, lesquelles sont différentes de l'apolipoprotéine constituant le premier sous-ensemble et différente des éventuelles autres apolipoprotéines portées par les particules lipoprotéiques du premier groupe), pour que la hauteur du pic obtenue soit $H_2$, cette détermination ayant lieu en mettant sur la plaque une quantité connue d'anticorps $v_2$ correspondant à la précipitation des particules lipoprotéiques du deuxième groupe, ce qui donne une hauteur $h_2$, la quantité $V_2$ étant alors calculée selon la formule suivante :

$$V_2 = \frac{h_2 v_2}{H_2}$$

Lorsqu'on utilise la technique d'immunodiffusion radiale on a intérêt à ce que le diamètre de l'anneau soit le plus grand possible étant donné que l'erreur relative sur sa mesure est d'autant plus faible que celui-ci est plus grand.

Pour profiter au mieux des dimensions de la plaque, on procède comme suit.

On détermine :

1°) la quantité d'anticorps $v_1$ (destinés à précipiter les particules lipoprotéiques portant l'apolipoprotéine du premier sous-ensemble, laquelle apolipoprotéine est également portée par une partie au moins des apolipoprotéines du deuxième groupe), pour que le diamètre de l'anneau obtenu soit $D_1$, cette détermination ayant lieu en mettant sur la plaque une quantité connue d'anticorps $v_1$ correspondant à la précipitation des particules lipoprotéiques du premier groupe, ce qui donne un diamètre $d_1$, la quantité $V_1$ étant alors calculée selon la formule suivante :

$$V_1 = x\%v_1 \;\frac{d_1^2}{D_1^2}$$

x% représentant le pourcentage de l'apolipoprotéine constituant le premier sous-ensemble, par rapport à la quantité totale de cette apolipoprotéine contenue dans le milieu,

et on détermine

2°) la quantité d'anticorps $V_2$ (destinés à précipiter les particules lipoprotéiques portant la ou les apolipoprotéines du deuxième sous-ensemble, lesquelles sont différentes de l'apolipoprotéine constituant le premier sous ensemble et différentes des éventuelles autres apolipoprotéines portées par les particules lipoprotéiques du premier groupe), pour que le diamètre de l'anneau obtenu soit $D_2$, cette détermination ayant lieu en mettant sur la plaque une quantité connue d'anticorps $v_2$ correspondant à la

précipitation des apolipoprotéines du deuxième sous-ensemble, ce qui donne un rayon $d_2$, la quantité $V_2$ étant alors calculée selon la formule suivante:

$$V_2 = v_2 \frac{d_2^{\,2}}{D_2^{\,2}}$$

Selon un mode de réalisation préféré du procédé de l'invention, le premier sous-ensemble est constitué par l'apolipoprotéine A I, portée par les particules lipoprotéiques LP AI et LP AI:E, constituant le premier groupe et le deuxième sous-ensemble est constitué par l'apolipoprotéine A II, portée par les particules lipoprotéiques LP AI:A II et LPE:A II, constituant le deuxième groupe, caractérisé en ce que l'on met le milieu contenant les deux sous-ensembles à doser en présence d'un mélange d'anticorps respectivement dirigés contre l'apolipoprotéine A II (anticorps anti A II), et contre l'apolipoprotéine A I (anticorps anti A I), dans des conditions telles que les anticorps dirigés contre l'apolipoprotéine A II soient en excès par rapport aux anticorps dirigés contre l'apolipoprotéine A I, cet excès correspondant notamment au fait que le produit de la valeur du volume d'anticorps anti A II mis sur la plaque par la valeur du titre des anticorps anti A II, est supérieur au produit de la valeur du volume d'anticorps anti A I mis sur la plaque par la valeur du titre des anticorps anti A I, parce que pour un sujet normal, la quantité d'apolipoprotéine A1 portée par les particules LPAI et LPAI:E est sensiblement la même que la quantité d'apolipoprotéines $A_2$ totale).

Selon un autre mode de réalisation préféré du procédé de l'invention, le premier sous-ensemble est constitué par l'apolipoprotéine B, portée par les particules lipoprotéiques LPB, constituant le premier groupe et le deuxième sous-ensemble est constitué par les deux apolipoprotéines E et C III, portées par les particules lipoprotéiques LPB:E, LPB:CIII, LPE:CI:CII:CIII:B, LPAI:E et LPE:A II constituant le deuxième groupe, caractérisé en ce que l'on met le milieu contenant les deux sous-ensembles à doser en présence d'un mélange d'anticorps respectivement dirigés contre l'apolipoprotéine E (anticorps anti E), l'apolipoprotéine C III (anticorps anti CIII) et l'apolipoprotéine B (anticorps anti B), dans des conditions telles que, d'une part, les anticorps dirigés contre l'apolipoprotéine E soient en excès par rapport aux anticorps dirigés contre l'apolipoprotéine B, et, d'autre part, les anticorps dirigés contre l'apolipoprotéine C III soient en excès par rapport aux anticorps dirigés contre l'apolipoprotéine B, l'excès des anticorps anti C III par rapport aux anticorps anti B pouvant être exprimé par le fait que le produit de la valeur du volume des anticorps anti C III par la valeur du titre des anticorps anti C III soit supérieur au sixième du produit de la valeur du volume des anticorps anti B par la valeur du titre des anticorps anti B, et l'excès des anticorps anti E par rapport aux anticorps anti B pouvant être exprimés par le fait que le produit de la valeur du volume d'anticorps anti E par la valeur du titre des anticorps anti E soit supérieur au douzième du produit de la valeur du volume des anticorps anti B par la valeur du titre des anticorps anti B.

Selon un autre mode de réalisation préféré du procédé de l'invention, on dose trois sous-ensembles d'apolipoprotéines appartenant respectivement à trois groupes de particules lipoprotéiques, le premier sous-ensemble étant constitué par l'apolipoprotéine B, appartenant aux particules lipoprotéiques LPB constituant le premier groupe, le deuxième sous-ensemble étant constitué par l'apolipoprotéine E, appartenant aux particules lipoprotéiques LPB:E constituant le deuxième groupe, et le troisième sous-ensemble étant constitué par les apolipoprotéines A I, A II et C III appartenant aux particules lipoprotéiques LPE:CI:CII: CIII:B, LPB:CIII, LPE: AII, LPAI:E, LPA I et LP AI:AII, constituant le troisième groupe, caractérisé en ce qu'on met le milieu contenant les trois sous-ensembles à doser respectivement en présence d'un mélange d'anticorps dirigés contre l'apolipoprotéine C III (anticorps anti C III), contre l'apolipoprotéine A II (anticorps anti A II), contre l'apolipoprotéine A I (anticorps anti A I), contre l'apolipoprotéine E (anticorps anti E) et contre l'apolipoprotéine B (anticorps anti B), les anticorps anti C III, anti A II et anti A I étant respectivement en excès par rapport aux anticorps anti E et anti B, et les anticorps anti E étant en excès par rapport aux anticorps anti B.

Selon un autre mode de réalisation préféré du procédé de l'invention, on dose trois sous-ensembles d'apolipoprotéines appartenant respectivement à trois groupes de particules lipoprotéiques, le premier sous-ensemble étant constitué par l'apolipoprotéine A I, appartenant aux particules lipoprotéiques LPAI constituant le premier groupe, le deuxième sous-ensemble étant constitué par l'apolipoprotéine A II, appartenant aux particules lipoprotéiques LPAI:AII constituant le deuxième groupe, et le troisième sous-ensemble étant constitué par l'apolipoprotéine E, appartenant aux particules lipoprotéiques LPE:CI:CII: CIII:B, LPB:E, LPE:AII et LPAI:E, constituant le troisième groupe, caractérisé en ce qu on met le milieu contenant les trois sous-ensembles à doser respectivement en présence d'un mélange d'anticorps dirigés contre l'apolipoprotéine E

(anticorps anti E), contre l'apolipoprotéine A II (anticorps anti A II) et contre l'apolipoprotéine A I (anticorps anti A I), les anticorps anti E étant respectivement en excès par rapport aux anticorps anti A II et anti A I, et les anticorps anti A II étant en excès par rapport aux anticorps anti E.

La présente invention a encore pour objet l'application de ce nouveau procédé de dosage au dépistage in vitro de maladies, notamment corrélées à des teneurs en apolipoprotéines situées à l'extérieur du domaine délimité par les valeurs extrêmes correspondant généralement à l'état physiologique d'un être humain ou animal sain.

L'invention concerne plus particulièrement des procédés de dépistage in vitro des risques d'athérosclérose corrélés à des teneurs en apolipoprotéine AI, ou en apolipoprotéine CII ou en apolipoprotéine CIII, situées à l'extérieur du domaine délimité par les valeurs extrêmes des susdites protéines correspondant à l'état physiologique d'un individu sain.

On rappelle que la constitution des différentes particules lipoprotéiques d'un sérum humain peut être schématisé comme suit

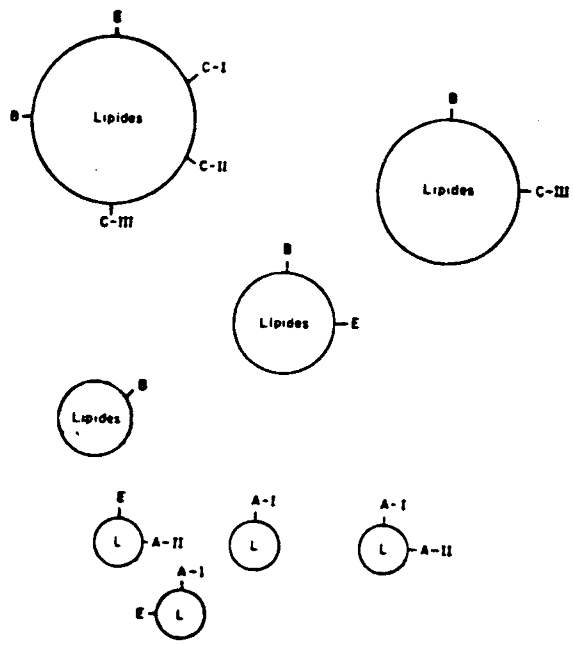

(cf. Fruchart - Ann. Biol. Clin. 1986-44-116-121).

On rappelle ci-après la concentration des valeurs physiopathologiques habituelles des principales apolipoprotéines dans le sérum des sujets adultes.

| Apolipoprotéines | |
|---|---|
| Apo A-I | 1,10 à 1,60 g/l |
| Apo A-II | 0,27 à 0,49 g/l |
| Apo $B_{100}$ | 0,7 à 1,3 g/l |
| Apo $B_{48}$ | concentration négligeable |
| Apo C-I | 50 à 110 mg/l |
| Apo C-II | 10 à 67 mg/l |
| Apo C-III | 40 à 140 mg/l |
| Apo E | 35 à 73 mg/l |
| Apo(a) | 0,01 à 2 500 mg/l |

L'invention concerne avantageusement un procédé de dépistage in vitro des risques d'athérosclérose corrélés à des teneurs en apolipoprotéines AI portés par les particules lipoprotéiques LPAI et LPAI:E situées à l'extérieur du domaine délimité par les valeurs extrêmes des susdites apolipoprotéines correspondant à l'état physiologique d'un individu sain, en mettant en oeuvre le procédé de dosage selon l'invention.

Il a en effet été démontré que le dosage des particules LPAI:E et LPAI était plus significatif que le

dosage de l'apolipoprotéine AI totale.

En effet, ces particules peuvent pénétrer dans les cellules et fixer le cholestérol pour l'amener au foie où il sera métabolisé.

Par contre, les particules LPAI:AII ne pénètrent pas dans les cellules et seraient même inhibitrices de la pénétration des LPAI et LPAI:E.

L'invention concerne avantageusement un procédé de dépistage in vitro des risques d'athérosclérose corrélés à des teneurs en apolipoprotéines B, portés par les particules lipoprotéiques LPB, situées à l'extérieur du domaine délimité par les valeurs extrêmes des susdites apolipoprotéines correspondant à l'état physiologique d'un individu sain, en mettant en oeuvre le procédé de dosage selon l'invention.

Il a également été démontré que le dosage de la particule LPB est plus représentatif des risques d'athérosclérose que le dosage de l'apolipoprotéine B totale.

L'invention concerne également des supports plans, notamment films ou plaques pour le dosage, prêts à l'utilisation, ainsi que pour le dépistage in vitro de risques pathologiques corrélés à des teneurs anormales en apolipoprotéines contenant un mélange d'anticorps correspondant respectivement à l'obtention du domaine de précipitation des groupes de particules lipoprotéiques portant les sous ensembles de particules à doser.

L'invention concerne également des nécessaires ou kits pour le dosage de sous-ensembles d'apolipoprotéines, ainsi que pour le dépistage in vitro des risques pathologiques corrélés à des teneurs anormales en apolipoprotéine qui comprennent

- un support plan, tel qu'un film ou une plaque de gel contenant un mélange d'anticorps correspondant à la précipitation des particules lipoprotéiques contenant respectivement les sous-ensembles d'apolipoprotéines à doser,
- une courbe d'étalonnage ou de préférence une solution biologique étalon de préférence à plusieurs dilutions déterminées, avantageusement 3 ou 4 dilutions déterminées contenant une quantité connue de sous-ensembles d'apolipoprotéines que l'on veut doser par ailleurs dans un milieu biologique.

En effet, pour faire les dosages de sous-ensembles d'apolipoprotéines contenues dans un milieu biologique, on peut avoir recours à une courbe d'étalonnage préalablement établie à partir d'échantillons étalon contenant des quantités déterminées des sous-ensembles d'apolipoprotéines, que l'on veut doser par ailleurs dans un milieu biologique.

Il est avantageux d'établir pour chaque film ou plaque de gel, une courbe d'étalonnage et pour ce faire, de disposer de solutions étalon contenant des quantités connues des sous-ensembles d'apolipoprotéines que l'on veut doser par ailleurs dans un milieu biologique en raison des légères variations qui peuvent intervenir lors de l'analyse proprement dite.

On donne ci-après un exemple de réalisation relatif aux dosages des deux sous ensembles des apolipoprotéines AI et AII, le premier sous-ensemble étant constitué par l'apolipoprotéine AI, portée par les particules LPAI:E et LPAI, formant le premier groupe et le deuxième sous-ensemble étant constitué par l'apolipoprotéine AII, portée par les particules LPE:AII et LPAI:AII.

Les essais sont effectués sur des plaques de gel de dimensions 10,3 x 8,3 cm avec une rangée de réservoirs (lieux de dépôt du milieu biologique) disposée à 1,7 cm de la grande dimension et parallélement à celle-ci, 1 cm de chaque côté étant occupé par les ponts de papier. Il reste 5,5 cm de "hauteur utile" au dessus des réservoirs.

On fixe arbitrairement pour un échantillon normal de milieu biologique, une hauteur de 3,5 cm pour le pic correspondant à la précipitation des particules lipoprotéiques LPAI:E et LPAI et une hauteur de 1,5 cm pour le pic correspondant à la précipitation des particules lipoprotéiques LPE:AII et LPAI:AII.

Ces valeurs arbitraires de hauteur de pic souhaitable, sont données à titre d'exemple pour montrer la manière dont on peut déterminer les quantités d'anticorps Anti A I et Anti A II, et ne sont en rien limitatives de la technique (dans la mesure où le pic correspondant à la précipitation des particules lipoprotéiques LPE:AII et LPAI:AII reste inférieur au pic correspondant à la précipitation des particules lipoprotéiques LPAI:E et LPAI).

On détermine tout d'abord la quantité d'anticorps Anti A II à incorporer au gel pour qu un échantillon normal de milieu biologique donne un pic de hauteur 1,5 cm.

L'échantillon est déposé dans une plaque contenant le volume v2 d'anticorps Anti A II. Après migration, on obtient un pic de hauteur "h". La quantité d'anticorps Anti A II à incorporer sera :

$$V2 = \frac{h}{1,5} v2$$

La détermination de la quantité d'anticorps Anti A I se fait de la même manière en tenant compte en plus du fait que, dans l'échantillon biologique susmentionné, la proportion d'antigène A I portée par les particules lipoprotéiques LPAI:E et LPAI représente pour un échantillon normal environ 25 % de la quantité totale d'antigène A I.

Si, dans une plaque contenant un volume v1 d'anticorps Anti A I, on obtient un pic de hauteur "h'", la quantité d'anticorps Anti A I à ajouter à la plaque contenant un volume V2 d'anticorps Anti A II sera :

$$V1 = 0,25 \frac{h'}{3,5} v1$$

Dans cette plaque contenant un volume V1 d'anticorps Anti A I et un volume V2 d'anticorps Anti II, l'échantillon donnera deux pics :

. un pic dont la frontière est la plus proche du point de dépôt de l'échantillon et dont la hauteur est de 1,5 cm (premier pic),
. un pic dont la frontière est la plus éloignée du point de dépôt de l'échantillon et dont la hauteur est de 3,5 cm (deuxième pic).

On peut vérifier que la hauteur du premier pic est indépendante de la quantité d'anticorps Anti A II présente dans le gel (dans la mesure où le deuxième pic reste inférieur au premier pic).

Ainsi, si dans un autre gel on incorpore un volume V1 d'anticorps Anti A I et deux volumes V2 d'anticorps Anti A II, on obtient un premier pic de même hauteur et un deuxième pic dont la hauteur est égale à la moitié de celle du pic obtenu avec le volume V2.

On a représenté de façon schématique sur la figure 1, une plaque sur laquelle on a effectué le dosage des deux sous-ensembles d'apolipoprotéines AI et AII, comme défini ci-dessus.

La plaque de la figure 1 contient un mélange de 0,8 ml d'anticorps anti $A_{II}$ (de titre 50) et 0,15 ml d'anticorps anti $A_{I}$ (de titre 175).

Les deux premiers pics de gauche et les deux derniers pics de droite correspondent à ceux obtenus avec un sérum étalon, à la dilution $\frac{1}{20}$ (mentionné par $\frac{SE}{20}$ )

On a fait des essais sur 12 échantillons de sérum dans lesquels on veut doser les sous-ensembles d'apolipoprotéines A-I et A-II, comme définis ci-dessus. Ces 12 échantillons sont testés à la dilution 1/20.

La figure 2 correspond à une plaque contenant un mélange d'anticorps Anti A-I et Anti A-II respectivement de même titre que dans la plaque n° 1, et dans laquelle le volume d'anticorps Anti A-I est le même que celui incorporé dans la plaque de la figure 1, mais dans lequel le volume d'anticorps Anti A-II est le double de celui incorporé dans la plaque de la figure 1.

On constate que les pics correspondent à la précipitation, pour chacun des sérums testés, des particules lipoprotéiques du premier groupe sont inchangés tandis que les pics correspondant à la précipitation des particules lipoprotéiques du deuxième groupe ont une hauteur égale à la moitié de la hauteur des pics du deuxième groupe de la première plaque.

Pour vérifier la validité du procédé de dosage de l'invention, on peut montrer que le premier pic ne comporte pas d'apolipoprotéine du deuxième sous-ensemble, tandis que le premier et le deuxième pics comportent l'apolipoprotéine du premier sous-ensemble.

Pour ce faire, on utilise trois plaques de gel, dans lesquelles on introduit un mélange de 0,15 ml d'anticorps Anti AI (titre 220) et de 1 ml d'anticorps Anti AII (titre 60).

Les trois premiers pics de gauche correspondent à un sérum étalon dilué, de gauche à droite respectivement, au 1/20, au 1/10 et 1/5e et les trois derniers pics de droite correspondent à un sérum étalon dilué de droite à gauche respectivement au 1/20, au 1/10 et au 1/5.

Les dix autres pics correspondent à des sérums à doser, dilués au 1/10e.

Après migration, une des plaques est lavée avec de l'eau physiologique, puis séchée et colorée avec un colorant des protéines, tel que le bleu de Coomassie. On obtient deux pics. C'est ce qui est représenté sur la plaque de la figure 3.

La figure 4 correspond à l'une des autres plaques parmi les trois plaques définies ci-dessus, incubée, après migration, avec une solution d'anticorps monoclonal anti A-II, à raison de 300 µl, marqué à la péroxydase.

La révélation est obtenue à l'aide d'un substrat chromogénique de la péroxydase et seul le pic correspondant à la précipitation des particules lipoprotéiques du deuxième groupe est observé.

La figure 5 correspond à la dernière plaque, incubée, après migration avec une solution d'anticorps monoclonal anti A-I, à raison de 400 µl. La révélation est obtenue à l'aide d'un substrat chromogénique de la péroxydase et les deux pics correspondant respectivement à la précipitation des particules lipoprotéiques du premier et du deuxième groupe sont observés.

On a également réalisé 3 plaques, d'immunodiffusion radiale. Chaque plaque contient un mélange de 1 ml d'anticorps anti A-II de titre (60), et de 0,12 ml d'anticorps anti A-I de titre (220).

La figure 6 correspond à la coloration d'une plaque à l'aide de bleu de Coomassie, ce qui donne 2 anneaux.

La figure 7 correspond à la révélation d'une des 3 plaques, incubée avec 300 µl d'anticorps anti A-II monoclonal marqué à la péroxydase, et révélée à l'aide d'un substrat chromogène de la péroxydase. On révèle ainsi l'anneau correspondant uniquement à la précipitation du deuxième groupe de particules lipoprotéiques.

La figure 8 correspond à la révélation d'une des 3 plaques incubée avec 400 µl d'anticorps anti A-I monoclonal marqué à la péroxydase et révélée à l'aide d'un substrat chromogène de la péroxydase. On révèle ainsi les anneaux correspondant à la précipitation du premier et du deuxième groupe de particules lipoprotéiques.

Exemple 1 :

Dans un erlenmeyer de 50 ml, on introduit 29 ml d'eau déminéralisée. On ajoute 250 mg d'agarose de marque HGT (commercialisé par la Société MCI) et 60 mg d'agarose de marque HEEO (agarose de haute électroendosmose commercialisé par MCI). On chauffe à 95-100°C jusqu'à l'obtention d'une solution parfaitement limpide. On l'équilibre alors à 52-55°C dans un bain thermostaté et on y ajoute 3 ml de tampon Tris Glycine, Veronal, Veronal-Na pH 9,2 de force ionique 0,1 (conductivité du tampon), puis 4 ml d'anticorps anti A-II (titre 60) et 0,6 ml d'anticorps anti A-I (titre 220).

La solution homogénéisée est coulée sur un film plastique de marque Gel bond® (commercialisé par FMC) de dimension 10,1 x 8,3 cm à raison de 9 ml/film. Une fois le gel formé, on découpe à l'emporte pièce sur une ligne parallèle à la longueur 16 puits de 3 mm de diamètre.

On obtient ainsi un film prêt à l'emploi pour le dosage de l'apolipoprotéine AI (portée par les particules lipoprotéiques LPAI:E et LPAI) et de l'apolipoprotéine A II (portée par les particules lipoprotéiques LPAI:AII et LPE:AII), par technique d'électroimmunodiffusion.

Les échantillons à doser, ainsi que la gamme des standards, sont déposés à la dilution au 1/10 à raison de 3 µl/puits. Après 3 heures de migration sous 150 volts et lavage en eau physiologique, séchage et coloration au bleu de Coomassie, on obtient, pour chaque échantillon, 2 pics dont le plus grand et le moins coloré correspond à la l'apolipoprotéine A I (portée par les particules lipoprotéiques LPAI:E et LPAI), le plus petit et le plus coloré correspond à l'apolipoprotéine A II (portée par les particules lipoprotéiques LPAI:AII et LPE:AII).

Exemple 2 :

On opère comme à l'exemple 1, à la différence que l'on ajoute 6 ml d'anticorps anti A-II (titre 60). Après migration des mêmes échantillons que ceux de l'exemple 1, on obtient un pic correspondant à l'apolipoprotéine A I (portée par les particules lipoprotéiques LPAI:E et LPAI) de même hauteur qu'à l'exemple 1 et un pic correspondant à l'apolipoprotéine A II (portée par les particules lipoprotéiques LPAI:AII et LPE:AII) et de hauteur divisée 1,5 par rapport à l'exemple 1.

Exemple 3 :

On opère comme à l'exemple 1, à la différence que l'on ajoute 4 ml d'anticorps anti A-II de titre 120 et 0,6 ml d'anticorps anti A-I de titre 330.

Après migration, les mêmes échantillons qu'à l'exemple 1 donnent un pic correspondant à l'apolipoprotéine A I (portée par les particules lipoprotéiques LPAI:E et LPAI) en hauteur divisée par 1,5 par rapport à l'exemple 1 et un pic correspondant à l'apolipoprotéine A II (portée par les particules lipoprotéiques LPAI:AII

et LPE:AII) dont la hauteur est divisée par 2 par rapport à l'exemple 1.

Exemple 4 :

On opère comme à l'exemple 1. Après migration, le gel est recouvert pendant 3 heures d'un papier filtre imprégné d'une solution d'un anticorps monoclonal anti A-II, marqué à la peroxydase.

Après lavage en eau physiologique pendant une nuit, et révélation de l'activité peroxydasique, seul le petit pic est coloré qui correspond à l'apolipoprotéine A II (portée par les particules lipoprotéiques LPAI:AII et LPE:AII).

Exemple 5 :

On opère comme à l'exemple 3, mais le papier filtre est imprégné d'un anticorps monoclonal anti A-I, marqué à la peroxydase. La révélation de l'activité peroxydasique colore dans ce cas les deux pics correspondant respectivement à l'apolipoprotéine A I ((portée par les particules lipoprotéiques LPAI:E et LPAI) et à l'apolipoprotéine A II (portée par les particules lipoprotéiques LPAI:AII et LPE:AII).

Exemple 6 :

On opère comme à l'exemple 1, à la différence que l'on coule 12 ml par film. On découpe 12 puits de diamètre 3 mm équidistants et répartis sur l'ensemble de la surface du gel. On obtient ainsi un film prêt à l'emploi utilisable pour le dosage de l'apolipoprotéine A I (portée par les particules lipoprotéiques LPAI:E et LPAI) dans la technique d'immunodiffusion radiale.

5 µl des échantillons et des standards sont déposés à la dilution 1/10. Après 48 heures de diffusion, lavage en eau physiologique pendant une nuit, séchage et coloration au noir amide, on obtient pour chaque échantillon 2 anneaux, dont le plus grand et le moins coloré correspond à l'apolipoprotéine A I (portée par les particules lipoprotéiques LPAI:E et LPAI) et le plus petit et le plus coloré à l'apolipoprotéine A II ((portée par les particules lipoprotéiques LPAI:AII et LPE:AII).

Exemple 7 :

On opère comme à l'exemple 1 à la différence que l'on ajoute à la solution d'agarose 1ml d'anticorps Anti E (titre 60) 2,8 ml d'anticorps Anti CIII (titre 40) et 0,11 ml d'anticorps Anti B (titre 2000). On obtient ainsi un film prêt à l'emploi pour le dosage par immunodiffusion de l'apolipoprotéine B portée par la particule lipoprotéique LPB.

Exemple 8 :

On réalise une plaque déshydratée et réhydratable selon le brevet américain n° 2 086 541 du 01/04/70 et le brevet français n° 76 22802 du 27/07/76.

Dans un erlenmeyer de 50 ml, on introduit 30 ml d'eau déminéralisée et on ajoute sous agitation magnétiqique 1,8g de D-sorbitol, 90 mg de Separan Np10® commercialisé par la Société MCi (Marine Colloïds Incorporation) et 45 mg de Separan MGL® commercialisé par la Société MCi. Après solubilisation complète, on ajoute 250 mg d'agarose HGT et 70 mg d'agarose HEEO.

On chauffe la solution à 95-100°C jusqu'à solubilisation totale. Après avoir ramené la température à 53°C, on ajoute 4 ml d'anticorps anti A-II (titre 60) et 0,6 ml d'anticorps anti A-I (titre 220). La solution est répartie à raison de 9 ml/film. Après gelification, on découpe à l'emporte pièce 28 puits de diamètre 2 mm.

On place le film dans un flux d'air laminaire pendant une nuit. On obtient ainsi un film sec et réhydratable utilisable pour le dosage de l'apolipoprotéine A I (portée par les particules lipoprotéiques LPAI:E et LPAI) et de l'apolipoprotéine A II (portée par les particules lipoprotéiques LPAI:AII et LPE:AII).

Exemple 9 :

On opère comme à l'exemple 8, à la différence que l'on remplace les 250 mg HGT et les 70 mg HEEO par 300 mg d'agaragar et l'hétéropolymère basique par du Séparan CP35® commercialisé par la Société MCi. On répartit la solution à raison de 12 ml/film. On découpe 12 puits de 2,5 mm de diamètre équidistants et disposés sur l'ensemble de la surface du gel.

Après déshydratation, on obtient un film sec et réhydratable utilisable pour le dosage de l'apolipoproté-

ne A I (portée par les particules lipoprotéiques LPAI:E et LPAI) et de l'apolipoprotéine A II (portée par les particules lipoprotéiques LPAI:AII et LPE:AII) par technique d'immunodiffusion radiale.

**Revendications**

**1.** Procédé pour déterminer simultanément le contenu de chacun de deux ou plusieurs groupes de particules distinctes et indépendantes dans un mélange de particules au moyen d'immunodiffusion différentielle desdits groupes de particules sur un gel; lesdits groupes de particules comprenant :

1- un premier groupe de particules, dont chacune porte un premier antigène et

2- au moins un deuxième groupe de particules, dont chacune porte un second antigène et au moins certaines d'entre elles portent ledit premier antigène; ledit deuxième antigène n'étant pas porté par les particules du premier groupe, le procédé comprenant les étapes suivantes :

- traitement du mélange de particules avec un mélange d'anticorps comprenant un premier anticorps dirigé contre le premier antigène et un deuxième anticorps dirigé contre le deuxième antigène; ledit mélange d'anticorps contenant ledit premier anticorps en quantité au moins suffisante pour précipiter le premier groupe dans le mélange de particules; ledit mélange d'anticorps contenant ledit second anticorps en quantité au moins suffisante pour précipiter ledit second groupe dans le mélange de particules et en excès de ladite quantité du premier anticorps, ce par quoi les contenus respectifs de chaque premier et deuxième groupe dans ledit mélange de particules peut être déterminé.

**2.** Kit pour déterminer simultanément le contenu de chacun de deux ou plusieurs groupes de particules distinctes et indépendantes portant des antigènes dans un mélange de particules portant des antigènes, tels que définis à la revendication 1, ledit kit comprenant une plaque de gel et un mélange contenant des quantités prédéterminées de particules portant les premier et deuxième antigènes.

**3.** Procédé de dosage simultané par immunodiffusion différentielle sur gel en plaque d'au moins trois sous-ensembles d'apolipoprotéines, appartenant respectivement à des groupes de particules lipoprotéiques contenues dans un milieu biologique, ces trois groupes de particules lipoprotéiques étant tels qu'ils contiennent une "apolipoprotéine commune", et pouvant être définis de la façon suivante :

- l'un des groupes ci-après appelé premier groupe étant constitué par des particules lipoprotéiques portant toutes la susdite "apolipoprotéine commune", et portant d'éventuelles autres apolipoprotéines $X_m$, cette "apolipoprotéine commune" portée par toutes les particules lipoprotéiques du premier groupe constituant le premier sous-ensemble,
- un autre groupe, ci-après désigné par deuxième groupe ou groupe intermédiaire, étant tel que :
  - soit il est constitué de préférence par des particules lipoprotéiques portant toutes "l'apolipoprotéine commune" définie ci-dessus, et portant en outre au moins une apolipoprotéine $Y_n$ différente des éventuelles autres apolipoprotéines $X_m$ portées par les particules lipoprotéiques du premier groupe, cette ou ces apolipoprotéine(s) $Y_n$ constituant le deuxième sous-ensemble ou le sous-ensemble intermédiaire,
  - soit il est constitué par des particules lipoprotéiques dont d'une part une partie porte la susdite "apolipoprotéine commune", constituant le premier sous-ensemble, et porte, en outre, au moins une apolipoprotéine $Z_p$, différente des éventuelles autres apolipoprotéines $X_m$ portées par les particules lipoprotéiques du premier groupe, et dont, d'autre part, le reste des particules lipoprotéiques ne porte pas la susdite "apolipoprotéine commune", mais porte au moins une des apolipoprotéines $Z_p$ portées par la partie des particules lipoprotéiques susdéfinie et différentes des éventuelles autres apolipoprotéines $X_m$ portées par le premier groupe de particules lipoprotéiques, cette ou ces apolipoprotéine(s) $Z_p$ constituant le deuxième sous-ensemble, ou le sous-ensemble intermédiaire,
- le troisième groupe, ou dernier groupe, étant tel:
  - soit il est constitué par des particules lipoprotéiques contenant toutes "l'apolipoprotéine commune" définie ci-dessus et contenant en outre au moins une apolipoprotéine $W_q$, différente d'une part des éventuelles autres apolipoprotéines $X_m$ portées par les particules lipoprotéiques du premier groupe, et d'autre part des apolipoprotéines portées par les particules lipoprotéiques du deuxième groupe, cette ou ces apolipoprotéine(s) $W_q$ constituant le troisième sous-ensemble, ou dernier sous-ensemble ;
  - soit il est constitué par des particules lipoprotéiques dont, d'une part, une partie porte la susdite "apolipoprotéine commune" et porte en outre au moins une apolipoprotéine $T_1$

différente à la fois des éventuelles autres apolipoprotéines $X_m$ portées par les particules lipoprotéiques du premier groupe, et des apolipoprotéines portées par les particules lipoprotéiques du deuxième groupe, et dont, d'autre part, le reste des particules lipoprotéiques ne porte pas la susdite "apolipoprotéine commune", mais porte l'une au moins des apolipoprotéines $T_1$ portées par la partie des particules lipoprotéiques sus-définie, cette ou ces apolipoprotéines $T_1$ constituant le troisième sous-ensemble, ou dernier sous-ensemble ;

caractérisé en ce que :

- on met le milieu biologique contenant les sous-ensembles à doser en présence d'un mélange d'anticorps contenant
  . des anticorps dirigés contre la ou chacune des apolipoprotéines constituant le troisième ou dernier sous-ensemble en quantité suffisante pour précipiter les particules lipoprotéiques du troisième ou dernier groupe,
  . des anticorps dirigés contre la ou chacune des apolipoprotéines constituant le deuxième sous-ensemble, ou sous-ensemble intermédiaire, en quantité suffisante pour précipiter les particules lipoprotéiques du deuxième groupe, ou groupe intermédiaire ;
  . des anticorps dirigés contre "l'apolipoprotéine commune" aux sous-ensembles, et constituant le premier sous-ensemble, en quantité suffisante pour précipiter les particules lipoprotéiques du premier groupe ;
- la quantité des susdits anticorps nécessaires à la précipitation des particules lipoprotéiques du troisième ou dernier groupe étant en excès par rapport à la quantité des susdits anticorps nécessaires à la précipitation des particules lipoprotéiques du deuxième groupe ou groupe intermédiaire, cette dernière quantité des susdits anticorps nécessaires à la précipitation des particules lipoprotéiques du deuxième groupe ou groupe intermédiaire étant elle-même en excès par rapport à la quantité des susdits anticorps nécessaires à la précipitation des particules lipoprotéiques portant "l'apolipoprotéine commune" constituant le premier sous-ensemble, dans un rapport tel que, après immunodiffusion, on obtienne trois frontières distinctes, la frontière d'immunodiffusion la plus proche du point de dépôt du milieu contenant les sous-ensembles à doser, correspondant à la précipitation des particules lipoprotéiques du troisième ou dernier groupe, la frontière d'immunodiffusion la plus éloignée du susdit point de dépôt correspondant à la précipitation des particules lipoprotéiques du premier groupe, la frontière d'immunodiffusion intermédiaire entre la frontière la plus proche et la frontière la plus éloignée sus-mentionnées correspondant à la précipitation des particules lipoprotéiques du deuxième groupe ;
- on mesure les distances maximales respectives entre le point de dépôt et chacune des trois frontières d'immunodiffusion et on compare chacune des distances par rapport à celles obtenues avec un milieu étalon, contenant des quantités connues d'apolipoprotéines respectives des trois sous-ensembles.

**4.** Procédé selon la revendication 3 de dosage simultané par immunodiffusion différentielle sur gel en plaque d'au moins deux sous-ensembles d'apolipoprotéines, appartenant respectivement à au moins deux groupes de particules lipoprotéiques contenues dans un milieu biologique, ces deux groupes de particules lipoprotéiques étant tels qu'ils contiennent une "apolipoprotéine commune",

- l'un des groupes ci-après appelé premier groupe étant constitué par des particules lipoprotéiques portant toutes la susdite "apolipoprotéine commune", et portant d'éventuelles autres apolipoprotéines $X_m$, cette apolipoprotéine commune portée par toutes les particules lipoprotéiques du premier groupe constituant le premier sous-ensemble,
- l'autre groupe, ci-après désigné par deuxième groupe étant tel que :
  - soit il est constitué par des particules lipoprotéiques contenant toutes "l'apolipoprotéine commune" définie ci-dessus et portant en outre au moins une apolipoprotéine $Y_n$ différente des éventuelles autres apolipoprotéines $X_m$ portées par les particules lipoprotéiques du premier groupe, cette ou ces apolipoprotéine(s) $Y_n$ constituant le deuxième sous-ensemble ;
  - soit il est constitué par des particules lipoprotéiques dont, d'une part, une partie porte la susdite "apolipoprotéine commune" constituant le premier sous-ensemble et porte, en outre, au moins une apolipoprotéine $Z_p$ différente des éventuelles autres apolipoprotéines $X_m$ portées par les particules lipoprotéiques du premier groupe et dont, d'autre part, le reste des particules lipoprotéiques ne porte pas la susdite "apolipoprotéine commune" constituant le premier sous-ensemble mais porte l'une au moins des apolipoprotéines $Z_p$ portées par la partie des particules lipoprotéiques sus-définie et différente des éventuelles autres apolipoprotéines $X_m$

portées par le premier groupe de particules lipoprotéiques, cette ou ces apolipoprotéines $Z_p$ constituant le deuxième sous-ensemble ;
caractérisé en ce que :

- on met le milieu biologique contenant les deux sous-ensembles d'apolipoprotéine à doser en présence d'un mélange d'anticorps contenant
  . d'une part des anticorps dirigés contre la ou chacune des apolipoprotéines constituant le deuxième sous-ensemble ($Y_n$ ou $Z_p$) en quantité suffisante pour précipiter les particules lipoprotéiques du deuxième groupe ;
  . d'autre part des anticorps dirigés contre la susdite "apolipoprotéine commune", constituant le premier sous-ensemble, en quantité suffisante pour précipiter les particules lipoprotéiques du premier groupe ;
- la quantité des susdits anticorps nécessaires à la précipitation des particules lipoprotéiques du deuxième groupe étant en excès par rapport à la quantité des susdits anticorps nécessaires à la précipitation des particules lipoprotéiques du premier groupe, dans un rapport tel que, après immunodiffusion, on obtienne deux frontières distinctes, la frontière d'immunodiffusion la plus proche du point de dépôt du milieu contenant les deux sous-ensembles à doser correspondant à la précipitation des particules lipoprotéiques du deuxième groupe et la frontière d'immunodiffusion la plus éloignée du susdit point de dépôt correspondant à la précipitation des particules lipoprotéiques du premier groupe, ces deux frontières étant respectivement identiques à celles obtenues dans les conditions où, après avoir séparé les deux groupes de particules lipoprotéiques contenant respectivement le premier et le deuxième sous-ensembles d'apolipoprotéines, on met, d'une part, le premier groupe de particules lipoprotéiques en contact avec des anticorps dirigés contre l'apolipoprotéine commune constituant le premier sous-ensemble défini ci-dessus, en quantité identique à celle utilisée ci-dessus pour précipiter les particules lipoprotéiques du premier groupe, non séparées du milieu biologique et portant l'apolipoprotéine constituant le premier sous-ensemble, et on met, d'autre part, le deuxième groupe de particules lipoprotéiques en contact avec des anticorps dirigés contre les apolipoprotéines constituant le deuxième sous-ensemble défini ci-dessus, en quantité identique à celle utilisée ci-dessus pour précipiter les particules lipoprotéiques du deuxième groupe, non séparées du milieu biologique et portant les apolipoprotéines constituant le deuxième sous-ensemble;
- on mesure les distances maximales respectives entre le point de dépôt et chacune des deux frontières d'immunodiffusion et on compare chacune des distances par rapport à celles obtenues avec un milieu biologique étalon, contenant des quantités connues d'apolipoprotéines respectives des deux sous-ensembles.

**5.** Procédé selon la revendication 3 de dosage simultané par immunodiffusion différentielle sur gel en plaque de deux sous-ensembles d'apolipoprotéine, appartenant respectivement à deux groupes de particules lipoprotéiques contenues dans un milieu biologique, ces deux groupes de particules lipoprotéiques étant tels qu'ils contiennent une "apolipoprotéine commune",

- l'un des groupes ci-après appelé premier groupe étant constitué par des particules lipoprotéiques portant toutes la susdite "apolipoprotéine commune", et portant d'éventuelles autres apolipoprotéines $X_m$, cette apolipoprotéine commune portée par toutes les particules lipoprotéiques du premier groupe constituant le premier sous-ensemble, et l'autre groupe, ci-après désigné par deuxième groupe étant tel que :
  - soit il est constitué par des particules lipoprotéiques contenant toutes "l'apolipoprotéine commune" définie ci-dessus et portant en outre au moins une apolipoprotéine $Y_n$ différente des éventuelles autres apolipoprotéines $X_m$ portées par les particules lipoprotéiques du premier groupe, cette ou ces apolipoprotéine(s) $Y_n$ constituant le deuxième sous-ensemble ;
  - soit il est constitué par des particules lipoprotéiques dont, d'une part, une partie porte la susdite "apolipoprotéine commune" du premier groupe et porte en outre au moins une apolipoprotéine $Z_p$ différente des éventuelles autres apolipoprotéines $X_m$ portées par les particules lipoprotéiques du premier groupe et dont, d'autre part, le reste des particules lipoprotéiques ne porte pas la susdite "apolipoprotéine commune" constituant le premier sous-ensemble, mais porte l'une au moins des apolipoprotéines $Z_p$ portées par la partie des particules lipoprotéiques sus-définie et différente des éventuelles autres apolipoprotéines $X_m$ portées par le premier groupe de particules lipoprotéiques, cette ou ces apolipoprotéines $Z_p$ constituant le deuxième sous-ensemble; caractérisé en ce que :

- on met le milieu biologique contenant les deux sous-ensembles d'apolipoprotéine à doser en présence d'un mélange d'anticorps contenant
  . d'une part des anticorps dirigés contre la ou chacune des apolipoprotéines du deuxième sous-ensemble ($Y_n$ ou $Z_p$) en quantité suffisante pour précipiter les particules lipoprotéiques du deuxième groupe ;
  . d'autre part des anticorps dirigés contre "l'apolipoprotéine commune" aux deux sous-ensembles en quantité suffisante pour précipiter les particules lipoprotéiques du premier groupe ;
- la quantité des susdits anticorps nécessaires à la précipitation des particules lipoprotéiques du deuxième groupe étant en exces par rapport à la quantité des susdits anticorps nécessaires à la précipitation des particules lipoprotéiques du premier groupe, dans un rapport tel que, après immunodiffusion, on obtienne deux frontières distinctes, la frontière d'immunodiffusion la plus proche du point de dépôt du milieu contenant les deux sous-ensembles à doser correspondant à la précipitation des particules lipoprotéiques du deuxième groupe et la frontière d'immunodiffusion la plus éloignée du susdit point de dépôt correspondant à la précipitation des particules lipoprotéiques du premier groupe, ces deux frontières étant respectivement identiques à celles obtenues dans les conditions où, après avoir séparé les deux groupes de particules lipoprotéiques contenant respectivement le premier et le deuxième sous-ensembles d'apolipoprotéines, on met, d'une part, le premier groupe de particules lipoprotéiques en contact avec des anticorps dirigés contre l'apolipoprotéine commune du premier groupe en quantité identique à celle utilisée ci-dessus pour précipiter les particules lipoprotéiques et non séparées du milieu biologique, portant l'apolipoprotéine du premier sous-ensemble, et on met, d'autre part, le deuxième groupe de particules lipoprotéiques en contact avec des anticorps dirigés contre la ou les apolipoprotéines du deuxième sous-ensemble, différente(s) de l'apolipoprotéine du premier sous-ensemble, et différente des éventuelles autres apolipoprotéines portées par les particules lipoprotéiques du premier groupe en quantité identique à celle utilisée ci-dessus pour précipiter les particules lipoprotéiques et non séparées du milieu biologique, portant les apolipoprotéines du deuxième sous-ensemble ;
- on mesure les distances maximales respectives entre le point de dépôt et chacune des deux frontières d'immunodiffusion et on compare chacune des distances par rapport à celles obtenues avec un milieu biologique étalon, contenant des quantités connues d'apolipoprotéines respectives des deux sous-ensembles.

**6.** Procédé de dosage selon la revendication 5, dans lequel l'apolipoprotéine du premier sous-ensemble appartient à des particules lipoprotéiques libres et identiques, constituant le premier groupe.

**7.** Procédé de dosage selon la revendication 5, dans lequel le premier groupe est constitué d'une part par des particules lipoprotéiques libres et identiques entre elles, portant l'apolipoprotéine commune constituant le premier sous-ensemble et, d'autre part, par des particules lipoprotéiques complexes, portant outre la susdite apolipoprotéine commune, au moins une apolipoprotéine différente de la susdite apolipoprotéine commune, et différente des apolipoprotéines constituant le deuxième sous-ensemble.

**8.** Procédé de dosage selon l'une quelconque des revendications 5 à 7, dans lequel le deuxième sous-ensemble est constitué par une apolipoprotéine différente de l'apolipoprotéine commune constituant le premier sous-ensemble et différente des éventuelles autres apolipoprotéines portées par les particules lipoprotéiques du premier groupe, laquelle apolipoprotéine est portée par des particules lipoprotéiques complexes, portant en outre toutes la susdite apolipoprotéine constituant le premier sous-ensemble.

**9.** Procédé de dosage selon l'une quelconque des revendications 5 à 7, dans lequel le deuxième sous-ensemble est constitué par au moins une apolipoprotéine différente de l'apolipoprotéine constituant le premier sous-ensemble et différente des éventuelles autres apolipoprotéines portées par les particules lipoprotéiques du premier groupe, laquelle apolipoprotéine est portée d'une part par des particules complexes, portant en outre l'apolipoprotéine constituant le premier sous-ensemble, et portée d'autre part par des particules lipoprotéiques simples ou complexes ne portant pas l'apolipoprotéine constituant le premier sous-ensemble.

**10.** Procédé de dosage selon l'une quelconque des revendications 3 à 9, dans lequel l'immunodiffusion

différentielle est effectuée par électroimmunodiffusion selon la méthode de Laurell, ce qui conduit à l'obtention d'au moins trois pics.

**11.** Procédé de dosage selon l'une quelconque des revendications 3 à 9 par immunodiffusion radiale selon la méthode de Mancini, ce qui conduit à l'obtention d'au moins trois anneaux.

**12.** Procédé de dosage selon l'une quelconque des revendications 5 à 11, dans lequel la quantité minimale d'anticorps dirigés contre la ou les apolipoprotéines du deuxième sous-ensemble à utiliser est déterminée dès l'apparition de deux frontières distinctes d'immunodiffusion et est avantageusement située dans une gamme dont la valeur minimale est déterminée dès l'apparition de deux frontières distinctes d'immunodiffusion et dont la valeur maximale est déterminée dès que les particules lipoprotéiques portant les apolipoprotéines du deuxième sous-ensemble ne diffusent plus et sont précipitées sur le point de dépôt du milieu biologique, la frontière d'immunodiffusion correspondant à la précipitation des particules lipoprotéiques portant l'apolipoprotéine du premier sous-ensemble n'étant pas modifiée lorsque la quantité d'anticorps dirigés contre la ou les apolipoprotéines du deuxième sous-ensemble est supérieure à la valeur minimum ci-dessus définie.

**13.** Procédé de dosage selon l'une quelconque des revendications 5 à 12, dans lequel la quantité d'anticorps dirigés contre la ou les apolipoprotéines du deuxième sous-ensemble est telle que la distance maximale entre le point de dépôt et la frontière d'immunodiffusion correspondant la précipitation des particules lipoprotéiques contenant les apolipoprotéines du deuxième sous-ensemble est d'environ 1/2 à environ 1/8, avantageusement d'environ 1/3 à environ 1/4, de la distance maximale entre le point de dépôt et la frontière d'immunodiffusion correspondant à la précipitation des particules lipoprotéiques contenant l'apolipoprotéine du premier sous-ensemble.

**14.** Procédé de dosage selon l'une quelconque des revendications 5 à 13, dans lequel l'étalonnage est effectué à partir d'un milieu dans lequel le premier sous-ensemble contient une quantité connue (A) d'apolipoprotéine, laquelle est commune aux deux groupes, le deuxième sous-ensemble contient une ou des quantités connues (B); d'apolipoprotéine(s) différente(s) de l'apolipoprotéine commune aux deux groupes et différente des éventuelles autres apolipoprotéines appartenant aux particules lipoprotéiques du premier groupe :

- en mettant sur la plaque des anticorps dirigés contre chacune des apolipoprotéines du deuxième sous-ensemble en quantité nécessaire pour précipiter les particules lipoprotéiques du deuxième groupe, chacune de ces quantités étant respectivement en excès par rapport aux anticorps nécessaires à précipiter les particules lipoprotéiques du premier groupe, l'excès en anticorps dirigés contre les apolipoprotéines du deuxième sous-ensemble par rapport aux anticorps dirigés contre l'apolipoprotéine du premier sous-ensemble pouvant être exprimé par la relation suivante :

$$V_{2i}T_{2i} \times \frac{B_i}{A} \quad \text{supérieur à } V_1T_1,$$

dans laquelle $V_1$, $T_1$ représentent respectivement le volume et le titre des anticorps dirigés contre l'apolipoprotéine du premier sous-ensemble et $V_{2i}$, $T_{2i}$ représentent respectivement les volumes et les titres des anticorps dirigés respectivement contre chacune des apolipoprotéines du deuxième sous-ensemble.

**15.** Procédé de dosage par électroimmunodiffusion selon l'une quelconque des revendications 5 à 10 et 12 à 14, dans lequel on détermine :

- la quantité d'anticorps $V_1$, destinés à précipiter les particules lipoprotéiques portant l'apolipoprotéine du premier sous-ensemble, laquelle apolipoprotéine est commune aux deux groupes, pour que la hauteur du pic obtenue soit $H_1$ en mettant sur la plaque une quantité connue d'anticorps $v_1$ correspondant à la précipitation des particules lipoprotéiques du premier groupe, ce qui donne une hauteur $h_1$, la quantité $V_1$ étant alors calculée selon la formule suivante :

$$V_1 = x\ \%\ \frac{h_1 v_1}{H_1}$$

x % représentant le pourcentage de l'apolipoprotéine commune aux deux groupes contenue dans le premier sous-ensemble, par rapport à la quantité totale de cette apolipoprotéine contenue dans le milieu, et on détermine

- la quantité d'anticorps $V_2$ destinés à précipiter les particules lipoprotéiques portant la ou les apolipoprotéines du deuxième sous-ensemble, lesquelles sont différentes de l'apolipoprotéine commune aux deux groupes et différente des éventuelles autres apolipoprotéines portées par les particules lipoprotéiques du premier groupe, pour que la hauteur du pic obtenue soit $H_2$, en mettant sur la plaque une quantité connue d'anticorps $v_2$ correspondant à la précipitation des particules lipoprotéiques du deuxième groupe, ce qui donne une hauteur $h_2$, la quantité $V_2$ étant alors calculée selon la formule suivante :

$$V_2 = \frac{h_2 v_2}{H_2}$$

**16.** Procédé de dosage par immunodiffusion radiale selon l'une quelconque des revendications 5 à 9 et 11 à 14, dans lequel on détermine :

- la quantité d'anticorps $V_1$ destinés à précipiter les particules lipoprotéiques portant l'apolipoprotéine du premier sous-ensemble, laquelle apolipoprotéine est commune aux deux groupes, pour que le diamètre de l'anneau obtenu soit $D_1$ en mettant dans la plaque une quantité connue d'anticorps $v_1$ correspondant à la précipitation des particules lipoprotéiques du premier groupe ce qui donne un diamètre $d_1$, la quantité $V_1$ étant alors calculée selon la formule suivante :

$$V_1 = x\ \%\ \frac{d_1^2}{D_1^2}\ v_1$$

x % représentant le pourcentage de l'apolipoprotéine commune aux deux groupes et contenue dans le premier sous-ensemble par rapport à la quantité totale de cette apolipoprotéine contenue dans le milieu,

- la quantité d'anticorps $V_2$ destinés à précipiter les particules lipoprotéiques portant la ou les apolipoprotéines du deuxième sous-ensemble, lesquelles sont différentes de l'apolipoprotéine commune aux deux groupes et différente des éventuelles autres apolipoprotéines portées par les particules lipoprotéiques du premier groupe, pour que le diamètre de l'anneau obtenu soit $D_2$, est déterminée en mettant dans la plaque une quantité connue d'anticorps $v_2$ correspondant à la précipitation des apolipoprotéines du deuxième sous-ensemble, ce qui donne un diamètre $d_2$, la quantité $V_2$ étant alors calculée selon la formule suivante :

$$V_2 = \frac{d_2^2 v_2}{d_2^2}$$

**17.** Procédé de dosage selon l'une quelconque des revendications 5 à 16, dans lequel le premier sous-ensemble est constitué par l'apolipoprotéine AI, portée par les particules lipoprotéiques LP AI et LP AI:E, constituant le premier groupe et le deuxième sous-ensemble est constitué par l'apolipoprotéine A II, portée par les particules lipoprotéiques LP AI:A II et LPE:A II, constituant le deuxième groupe, caractérisé en ce que l'on met le milieu contenant les deux sous-ensembles à doser en présence d'un mélange d'anticorps respectivement dirigés contre l'apolipoprotéine A II (anticorps anti A II), et contre l'apolipoprotéine A I (anticorps anti A I), dans des conditions telles que les anticorps dirigés contre l'apolipoprotéine A II soient en excès par rapport aux anticorps dirigés contre l'apolipoprotéine A I, cet excès correspondant notamment au fait que le produit de la valeur du volume d'anticorps anti A II mis sur la plaque par la valeur du titre des anticorps anti A II, est supérieur au produit de la valeur du volume d'anticorps anti A I mis sur la plaque par la valeur du titre des anticorps anti A I.

**18.** Procédé de dosage selon l'une quelconque des revendications 5 à 16, dans lequel le premier sous-ensemble est constitué par l'apolipoprotéine B porté par les particules lipoprotéiques LPB, constituant le premier groupe et le deuxième sous-ensemble est constitué par les deux apolipoprotéines E et C III, portées par les particules lipoprotéiques LPB:E, LPB:CIII, LPE:CI:CII:CIII:B, LPAI:E et LPE:A II constituant le deuxième groupe, caractérisé en ce que l'on met le milieu contenant les deux sous-ensembles à doser en présence d'un mélange d'anticorps respectivement dirigés contre l'apolipoprotéine E (anticorps anti E), l'apolipoprotéine C III (anticorps anti CIII) et l'apolipoprotéine B (anticorps anti B), dans des conditions telles que les anticorps dirigés contre l'apolipoprotéine E soient en excès par rapport aux anticorps dirigés contre l'apolipoprotéine B, et les anticorps dirigés contre l'apolipoprotéine C III soient en excès par rapport aux anticorps dirigés contre l'apolipoprotéine B, l'excès des anticorps anti C III par rapport aux anticorps anti B pouvant être exprimé par le fait que le produit de la valeur du volume d'anticorps anti C III par la valeur du titre des anticorps anti C III soit supérieur au sixième du produit de la valeur du volume des anticorps anti B par la valeur du titre des anticorps anti B, et l'excès des anticorps anti E par rapport aux anticorps anti B pouvant être exprimés par le fait que le produit de la valeur du volume d'anticorps anti E par la valeur du titre des anticorps anti E soit supérieur au douzième du produit de la valeur du volume des anticorps anti B par la valeur du titre des anticorps anti B.

**19.** Procédé de dosage selon la revendication 3, dans lequel on dose trois sous-ensembles d'apolipoprotéines appartenant respectivement à trois groupes de particules lipoprotéiques, le premier sous-ensemble étant constitué par l'apolipoprotéine B, appartenant aux particules lipoprotéiques LPB constituant le premier groupe, le deuxième sous-ensemble étant constitué par l'apolipoprotéine E, appartenant aux particules lipoprotéiques LPB:E constituant le deuxième groupe, et le troisième sous ensemble étant constitué par les apolipoprotéines A I, A II et C III appartenant aux particules lipoprotéiques LPE:CI:CII:CIII:B, LPB:CIII, LPE: AII, LPAI:E, LPA I et LP AI AII, constituant le troisième groupe, caractérisé en ce qu'on met le milieu contenant les trois sous-ensembles à doser respectivement en présence d'un mélange d'anticorps dirigés contre l'apolipoprotéine C III (anticorps anti C III), contre l'apolipoprotéine A II (anticorps anti A II), contre l'apolipoprotéine A I, contre l'apolipoprotéine E (anticorps anti E) et contre l'apolipoprotéine B (anticorps anti B), les anticorps anti C III, anti A II et anti A I étant respectivement en excès par rapport aux anticorps anti E et anti B, et les anticorps anti E étant en excès par rapport aux anticorps anti B.

**20.** Procédé de dépistage in vitro de maladies, notamment corrélées à des teneurs en apolipoprotéines situées à l'extérieur du domaine délimité par les valeurs extrêmes correspondant généralement à l'état physiologique d'un être humain ou animal sain, en mettant en oeuvre le procédé de dosage selon l'une quelconque des revendications 3 à 19.

**21.** Support, notamment plaque ou film recouvert de gel prêt à être utilisé pour la mise en oeuvre du procédé de dosage ou du procédé de dépistage, selon les revendications 3 à 20, caractérisé en ce qu'elle comporte un mélange d'anticorps correspondant respectivement à l'obtention du domaine de précipitation des particules lipoprotéiques portant les apolipoprotéines à doser.

**22.** Kit ou nécessaire de dosage contenant un support, notamment plaque ou film recouvert de gel, selon la revendication 21, et au moins une courbe d'étalonnage, préalablement établie à partir d'échantillons étalon contenant des quantités déterminées des sous-ensembles d'apolipoprotéine, que l'on veut doser par ailleurs dans un milieu biologique, ou de préférence une solution biologique étalon de préférence à plusieurs dilutions déterminées, avantageusement 3 ou 4 dilutions déterminées contenant une quantité connue de sous-ensembles d'apolipoprotéine que l'on veut doser par ailleurs dans un milieu biologique.

**Claims**

**1.** Procedure for the simultaneous determination of the contents of each of two or of several groups of distinct and independent particles in a mixture of particles by means of differential immunodiffusion of said groups of particles on a gel slab; said groups of particles comprising:
1- a first group of particles, of which each carries a first antigen and
2- at least a second group of particles, of which each carries a second antigen and at least some of them carry said first antigen; said second antigen being not carried by the particles of the first group, said process comprising the following steps:
- treating the mixture of the particles with a mixture of antibodies comprising a first antibody directed against the first antigen and a second antibody directed against the second antigen; said mixture of antibodies containing said first antibody in an amount at least sufficient to precipitate the first group in the mixture of particles; said mixture of antibodies containing said second antibody in an amount at least sufficient to precipitate said second group in the mixture of particles and in excess of said amount of the first antibody whereby the respective contents of each first and second group in said mixture of particles can be determined.

**2.** Kit for the simultaneous determination of the contents of each of two or several groups of distinct and independent particles carrying antigens in a mixture of particles carrying the antigens; such as defined in claim 1, said kit comprising a gel slab and a mixture containing predetermined amounts of particles carrying the first and second antigens.

**3.** Procedure for the simultaneous determination by differential immunodiffusion on a gel slab of at least three subsets of apolipoproteins belonging respectively to groups of lipoprotein particles contained in a biological medium, these three groups of lipoprotein particles being such that they contain a "common apolipoprotein" and being capable of being defined in the following manner:
- one of the groups called hereafter the first group being constituted by lipoprotein particles all carrying the above-mentioned "common apolipoportein", and carrying other possible apolipoproteins $X_m$, this "common apolipoprotein" carried by all of the lipoprotein particles of the first group constituting the first subset,
- another group, hereafter designated as the second or intermediate group, being such that:
  - either it is constituted preferably by lipoprotein particles all carrying the "common apolipoprotein" defined above, and carrying in addition at least one apolipoprotein $Y_n$ different from the other possible apolipoproteins $X_m$ carried by the lipoprotein particles of the first group, this or these apolipoprotein(s) $Y_n$ constituting the second subset or the intermediate subset,
  - or it is constituted by lipoprotein particles, a part of which carries, on the one hand, the above-mentioned "common apolipoprotein" constituting the first subset and carries, in addition, at least one apolipoprotein $Z_p$, different from the other possible apolipoproteins $X_m$ carried by the lipoprotein particles of the first group, and the remainder of the lipoprotein particles, on the other hand, does not carry the above-mentioned "common apolipoprotein", but carries at least one of the apolipoproteins $Z_p$ carried by that part of the lipoprotein particles defined above and different from the other possible apolipoproteins $X_m$ carried by the first group of lipoprotein particles, this or these apolipoprotein(s) $Z_p$ constituting the second subset or the intermediate subset,

- the third group or last group being such that:
  - either it is constituted by lipoprotein particles all containing the "common apolipoprotein" defined above and containing in addition at least one apolipoprotein $W_q$, different, on the one hand, from the other possible apolipoproteins $X_m$ carried by the lipoprotein particles of the first group and, on the other hand, and from the apolipoproteins carried by the lipoprotein particles of the second group, this or these apolipoprotein(s) $W_q$ constituting the third subset or last subset;
  - or it is constituted by lipoprotein particles, a part of which carries, on the one hand, the above-mentioned "common apolipoprotein" and carries in addition at least one apolipoprotein $T_1$ different from both the other possible apolipoproteins $X_m$ carried by the lipoprotein particles of the first group, and from the apolipoproteins carried by the lipoprotein particles of the second group, and the remainder of which lipoprotein particles, on the other hand, does not carry the above-mentioned "common apolipoprotein", but carries at least one of the apolipoproteins $T_1$ carried by that part of the lipoprotein particles defined above, this or these apolipoproteins $T_1$ constituting the third subset or last subset; characterized in that:
  - the biological medium containing the subsets to be determined is placed in the presence of a mixture of antibodies containing
    . antibodies directed against the apolipoprotein(s) constituting the third or last subset in an amount sufficient to precipitate the lipoprotein particles of the third or last group;
    . antibodies directed against the apolipoprotein(s) constituting the second subset, or intermediate subset, in an amount sufficient to precipitate the lipoprotein particles of the second group or intermediate group;
    . antibodies directed against the "common apolipoprotein" of the subsets, and constituting the first subset, in an amount sufficient to precipitate the lipoprotein particles of the first group;
  - the amount of the above-mentioned antibodies necessary for the precipitation of the lipoprotein particles of the third or last group being in excess with respect to the amount of the above-mentioned antibodies necessary for the precipitation of the lipoprotein particles of the second group or intermediate group, this latter quantity of the above-mentioned antibodies necessary for the precipitation of the lipoprotein particles of the second group or intermediate group being itself in excess with respect to the quantity of the above-mentioned antibodies necessary for the precipitation of the lipoprotein particles carrying the "common apolipoprotein" constituting the first subset, in a ratio such that, after immunodiffusion, three distinct lines are obtained, the immunodiffusion line closest to the site of deposition of the medium containing the subsets to be determined corresponding to the precipitation of the lipoprotein particles of the third or last group, the immunodiffusion line the furthest removed from the above-mentioned site of deposition corresponding to the precipitation of the lipoprotein particles of the first group, the immunodiffusion line intermediate between the above-mentioned closest line and line furthest removed corresponding to the precipitation of the lipoprotein particles of the second group;
  - the respective maximal distances between the site of deposition and each of the three immunodiffusion lines are measured and each of the distances is compared with those obtained with a standard medium containing known quantities of the respective apolipoproteins of the three subsets.

**4.** Procedure according to Claim 3, for the simultaneous determination by differential immunodiffusion on a gel plate of at least two subsets of apolipoproteins, belonging respectively to at least two groups of lipoprotein particles contained in a biological medium, these two groups of lipoprotein particles being such that they contain a "common apolipoprotein",

- one of the groups hereafter called the first group being constituted by lipoprotein particles all carrying the above-mentioned "common apolipoprotein", and carrying other possible apolipoproteins $X_m$, this common apolipoprotein carried by all of the lipoprotein particles of the first group constituting the first subset,
- the other group, hereafter designated as the second group being such that:
  - either it is constituted by lipoprotein particles all containing the "common apolipoprotein" defined above and carrying in addition at least one apolipoprotein $Y_n$ different from the other possible apolipoproteins $X_m$ carried by the lipoprotein particles of the first group, this or these apolipoprotein(s) $Y_n$ constituting the second subset;
  - or it is constituted by lipoprotein particles, a part of which carries, on the one hand, the above-mentioned "common apolipoprotein" constituting the first subset and carries, in addition, at least one apolipoprotein $Z_p$ different from the other possible apolipoproteins $X_m$ carried by the lipoprotein particles of the first group and the remainder of these lipoprotein particles, on the other hand, which do not carry the above-mentioned "common apolipoprotein" constituting the first subset but carry at least one of the apolipoproteins $Z_p$ carried by that part of the lipoprotein particles defined above and which is different from the other possible apolipoproteins $X_m$ carried by the first group of lipoprotein particles, this or these apolipoproteins $Z_p$ constituting the second subset;
characterized in that:

- the biological medium containing the two subsets of apolipoproteins to be determined is placed in the presence of a mixture of antibodies containing
  . on the one hand, antibodies directed against the apolipoprotein(s) constituting the second subset ($Y_n$ or $Z_p$) in sufficient quantity to precipitate the lipoprotein particles of the second group;
  . on the other hand, antibodies directed against the above-mentioned "common apolipoprotein", constituting the first subset, in sufficient quantity to precipitate the lipoprotein particles of the first group;
- the quantity of the above-mentioned antibodies necessary for the precipitation of the lipoprotein particles of the second group being in excess with respect to the quantity of the above-mentioned antibodies necessary for the precipitation of the lipoprotein particles of the first group, in a ratio such that, after immunodiffusion, two distinct lines are obtained, the immunodiffusion line closest to the site of deposition of the medium containing the two subsets to be determined corresponding to the precipitation of the lipoprotein particles of the second group and the immunodiffusion line the furthest removed from the above-mentioned site of deposition corresponding to the precipitation of the lipoprotein particles of the first group, these two lines being respectively identical to those obtained under the conditions in which, after the two groups of lipoprotein particles containing respectively the first and second subsets of apolipoproteins have been separated, the first group of lipoprotein particles, on the one hand, is placed in contact with antibodies directed against the common apolipoprotein constituting the first subset defined above in an amount identical with that used above to precipitate the lipoprotein particles of the first group unseparated from the biological medium and carrying the apolipoprotein constituting the first subset, and the second group of lipoprotein particles, on the other hand, is placed in contact with antibodies directed against the apolipoproteins constituting the second subset defined above in an amount identical with that used above to precipitate the lipoprotein particles of the second group, unseparated from the biological medium and carrying the apolipoproteins constituting the second subset;
- the respective maximal distances between the site of deposition and each of the two inmunodiffusion lines are measured and each of the distances is compared with those obtained with a standard biological medium containing known amounts of the respective apolipoproteins of the two subsets.

**5.** Procedure according to claim 3, for the simultaneous determination by differential immuno diffusion on a gel plate of two subsets of apolipoproteins belonging respectively to two groups of lipoprotein particles contained in a biological medium, these two groups of lipoprotein particles being such that they contain a "common apolipoprotein",

- one of the groups hereafter called the first group being constituted by lipoprotein particles all carrying the above-mentioned "common apolipoprotein", and carrying other possible apolipoproteins $X_m$, this common apolipoprotein carried by all of the lipoprotein particles of the first group constituting the first subset, and the other group, designated hereafter as the second group being such that:
  - either it is constituted by lipoprotein particles all containing the "common apolipoprotein" defined above and carrying in addition at least one apolipoprotein $Y_n$ different from the other possible apolipoproteins $X_m$ carried by the lipoprotein particles of the first group, this or these apolipoprotein(s) $Y_n$ constituting the second subset;
  - or it is constituted by lipoprotein particles, a part of which carries, on the one hand, the above-mentioned "common apolipoprotein" of the first group and carries in addition at least one apolipoprotein $Z_p$ different from the other possible apolipoproteins $X_m$ carried by the lipoprotein particles of the first group and the remainder of the lipoprotein particles which, on the other

hand, do not carry the above-mentioned "common apolipoprotein" constituting the first subset, but carry at least one of the apolipoproteins $Z_p$ carried by that part of the lipoprotein particles defined above and which is different from the other possible apolipoproteins $X_m$ carried by the first group of lipoprotein particles, this or these apolipoprotein(s) $Z_p$ constituting the second subset;

characterized in that,

- the biological medium containing the two subsets of apolipoproteins to be determined is placed in the presence of a mixture of antibodies containing
  . on the one hand, antibodies directed against the apolipoprotein or each of the apolipoproteins of the second subset ($Y_n$ or $Z_p$) in sufficient quantity to precipitate the lipoprotein particles of the second group;
  . on the other hand, antibodies directed against the "common apolipoprotein" of the two subsets in sufficient quantity to precipitate the lipoprotein particles of the first group;
- the quantity of the above-mentioned antibodies necessary for the precipitation of the lipoprotein particles of the second group being in excess with respect to the quantity of the above-mentioned antibodies necessary for the precipitation of the lipoprotein particles of the first group, in a ratio such that, after immunodiffusion, two distinct lines are obtained, the immunodiffusion line closest to the site of deposition of the medium containing the two subsets to be determined corresponding to the precipitation of the lipoprotein particles of the second group and the immunodiffusion line furthest removed from the above-mentioned site of deposition corresponding to the precipitation of the lipoprotein particles of the first group, these two lines being respectively identical with those obtained under the conditions in which, after the two groups of lipoprotein particles containing respectively the first and the second subsets of apolipoproteins have been separated, the first group of lipoprotein particles, on the one hand, is placed in contact with antibodies directed against the common apolipoprotein of the first group in an amount identical with that used above to precipitate the lipoprotein particles unseparated from the biological medium, carrying the apolipoprotein of the first subset, and, on the other hand, the second group of lipoprotein particles is placed in contact with antibodies directed against the apolipoprotein(s) of the second subset, different from the apolipoprotein of the first subset and different from the other possible apolipoproteins carried by the lipoprotein particles of the first group in an amount identical with that used above to precipitate the lipoprotein particles unseparated from the biological medium, carrying the apolipoproteins of the second subset;
- the respective maximal distances between the site of deposition and each of the two immunodiffusion lines are measured and each of the distances is compared with those obtained with a standard biological medium containing known amounts of the respective apolipoproteins of the two subsets.

**6.** Procedure for the determination according to claim 5, in which the apolipoprotein of the first subset belongs to free and identical lipoprotein particles, constituting the first group.

**7.** Procedure for the determination according to claim 5, in with the first group is constituted, on the one hand, by free and identical lipoprotein particles carrying the common apolipoprotein constituting the first subset and, on the other hand, by complex lipoprotein particles, carrying in addition to the above-mentioned common apolipoprotein, at least one apolipoprotein different from the above-mentioned common apolipoprotein, and different from the apolipoproteins constituting the second subset.

**8.** Procedure for the determination according to any one of the Claims 5 to 7, in which the second subset is constituted by an apolipoprotein different from the common apolipoprotein constituting the first subset and different from the other possible apolipoproteins carried by the lipoprotein particles of the first group, which apolipoprotein is carried by complex lipoprotein particles, all carrying in addition the above-mentioned apolipoprotein constituting the first subset.

**9.** Procedure for the determination according to any one of the Claims 5 to 7, in which the second subset is constituted by at least one apolipoprotein different from the apolipoprotein constituting the first subset and different from the other possible apolipoproteins carried by the lipoprotein particles of the first group, which apolipoprotein is carried, on the one hand, by complex particles, carrying in addition the apolipoprotein constituting the first subset, and is carried, on the other hand, by simple and

complex lipoprotein particles not carrying the apolipoprotein constituting the first subset.

**10.** Procedure for the determination according to any one of the Claims 3 to 9, in which the differential immunodiffusion is carried out by electroimmunodiffusion according to the method of Laurell, which gives rise to at least three peaks.

**11.** Procedure for the determination according to any one of the Claims 3 to 9 by radial immunodiffusion according to the method of Mancini, which gives rise to at least three rings.

**12.** Procedure for the determination acccording to any one of the Claims 5 to 11, in which the minimal amount of antibodies directed against the apolipoprotein(s) of the second subset to be used is determined from the first appearance of two distinct immunodiffusion lines and is advantageously located in a range, the minimal value of which is determined from the first appearance of two distinct immunodiffusion lines and the maximal value of which is that at which the lipoprotein particles carrying the apolipoproteins of the second subset no longer diffuse and are precipitated at the site of deposition of the biological medium, the immunodiffusion line corresponding to the precipitation of the lipoprotein particles carrying the apolipoprotein of the first subset being unaltered when the amount of antibodies directed against the apolipoprotein(s) of the second subset is greater than the minimal value defined above.

**13.** Procedure for the determination according to any one of the Claims 5 to 12, in which the amount of antibodies directed against the apolipoprotein(s) of the second subset is such that the maximal distance between the site of deposition and the immunodiffusion line corresponding to the precipitation of the lipoprotein particles containing the apolipoproteins of the second subset is about 1/2 to about 1/8, and advantageously about 1/3 to about 1/4, of the maximal distance between the site of deposition and the immunodiffusion line corresponding to the precipitation of the lipoprotein particles containing the apolipoprotein of the first subset.

**14.** Procedure for the determination according to any one of the Claims 5 to 13, in which the calibration is carried out starting from a medium in which the first subset contains a known amount (A) of apolipoprotein which is common to the two groups, the second subset contains one or more known quantities $(B)_i$ of apolipoprotein(s) different from the apolipoprotein common to the two groups and different from the other possible apolipoproteins belonging to the lipoprotein particles of the first group:

- by placing on the plate antibodies directed against each of the apolipoproteins of the second subset in quantities necessary to precipitate the lipoprotein particles of the second group, each of these quantities being respectively in excess with respect to the antibodies required to precipitate the lipoprotein particles of the first group, it being possible to express the excess of antibodies directed against the apolipoproteins of the second subset with respect to the antibodies directed against the apolipoprotein of the first subset by the following relationship:

$$V_{2i}T_{2i} \times \frac{B_i}{A} \text{ greater than } V_1T_1,$$

  in which $V_1$, $T_1$ represent respectively the volume and the titer of the antibodies directed against the apolipoprotein of the first subgroup and $V_{2i}$, $T_{2i}$ represent respectively the volumes and the titers of the antibodies directed respectively against each of the apolipoproteins of the second subset.

**15.** Procedure for the determination according to any one of the Claims 5 to 10, and 12 to 14, in which a determination is made of :

- the quantity of antibodies $V_1$, intended for the precipitation of the lipoprotein particles carrying the apolipoprotein of the first subset, which apolipoprotein is common to the two groups, to give a peak height of $H_1$ by placing on the plate a quantity of antibodies $V_1$ corresponding to the precipitation of the lipoprotein particles of the first group,which gives a height $h_1$, the quantity $V_1$ being then calculated from the following formula:

$$V_1 = x\,\%\ \frac{h_1 v_1}{H_1}$$

x% representing the percentage of the apolipoprotein common to the two groups contained in the first subset in relation to the total quantity of this apolipoprotein contained in the medium, and a determination is made of:

- the quantity of antibodies $V_2$ intended for the precipitation of the lipoprotein particles carrying the apolipoprotein(s) of the second subset, which are different from the apolipoproteins common to the two groups and different from the other possible apolipoproteins carried by the lipoprotein particles of the first group, to give a peak height of $H_2$ by placing on the plate a quantity of antibodies $v_2$ corresponding to the precipitation of the lipoprotein particles of the second group, which gives a height $h_2$, the quantity $V_2$ then being calculated from the following formula:

$$V_2 = \frac{h_2 v_2}{H_2}$$

**16.** Procedure for the determination according to any one fo the Claims 5 to 9, and 11 to 14, in which a determination is made of :

- the quantity of antibodies $V_1$ intended for the precipitation of the lipoprotein particles carrying the apolipoprotein of the first subset, which apolipoprotein is common to the two groups, to give a ring diameter of $D_1$ by placing in the plate a quantity of antibodies $v_1$ corresponding to the precipitation of the lipoprotein particles of the first group which gives a diameter $v_1$ of the lipoprotein particles of the first group which gives a diameter $d_1$, the quantity $V_1$ then being calculated from the following formula:

$$V_1 = x\%\ \frac{d_1^2}{D_1^2}\ v_1$$

x% representing the percentage of the apolipoprotein common to the two groups and contained in the first subset in relation to the total quantity of this apolipoprotein contained in the medium,

- the quantity of antibodies $V_2$ intended for the precipitation of the lipoprotein particles carrying the apolipoprotein(s) of the second subset, which are different from the apolipoprotein common to the two groups and different from the other possible apolipoproteins carried by the lipoprotein particles of the first group, to give a ring diameter of $D_2$, is determined by placing in the plate a quantity of antibodies $v_2$ corresponding to the precipitation of the apolipoproteins of the second subset, which gives a diameter $d_2$, the quantity $V_2$ then being calculated from the following formula:

$$V_2 = \frac{d_2^2 v_2}{d_2^2}$$

**17.** Procedure for the determination according to any one of the Claims 5 to 16, in which the first subset is constituted by the apolipoprotein AI, carried by the lipoprotein particles LP AI and LP AI:E, constituting

the first group and the second subset is constituted by the apolipoprotein A II, carried by the lipoprotein particles LP AI:A II and LPE:A II, contituting the second group, characterized in that the medium containing the two subsets to be determined is placed in the presence of a mixture of antibodies respectively directed against the apolipoprotein A II (anti A II antibodies) and against the apolipoprotein A I (anti A I antibodies) under conditions such that the antibodies directed against the apolipoprotein A II are in excess with respect to the antibodies directed against the apolipoprotein A I, this excess corresponding in particular to the fact that the product of the value for the volume of anti A II antibodies placed on the plate times the value for the titer of the anti A II antibodies is greater than the product of the value for the volume of anti A I antibodies placed on the plate times the value of the titer of the anti A I antibodies.

**18.** Procedure for the determination according to any one of the Claims 5 to 16, in which the first subset is constituted by the apolipoprotein B carried by the lipoprotein particles LPB, constituting the first group and the second subset is constituted by the two apolipoproteins E and C III, carried by the lipoprotein particles LPB:E LPB:CIII, LPE:CI:CII:CIII:B, LPAI:E and LPE:A II constituting the second group, characterized in that the medium containing the two subsets to be determined is placed in the presence of a mixture of antibodies respectively directed against the apolipoprotein E (anti E antibodies), the apolipoprotein C III (anti C III antibodies) and the apolipoprotein B (anti B antibodies) under conditions such that the antibodies directed against the apolipoprotein E are in excess with respect to the antibodies directed against the apolipoprotein B, it being possible to express the excess of the anti C III antibodies with respect to the anti B antibodies by the fact that the product of the value for the volume of anti C III antibodies times the value for the titer of the anti C III antibodies is six times greater than the product of the value for the volume of the anti B antibodies times the value for the titer of the anti B antibodies, and it being possible to express the excess of the anti E antibodies with respect to the anti B antibodies by the fact that the product of the value for the volume of anti E antibodies times the value for the titer of the anti E antibodies is 12 times greater than the product of the value for the volume of the anti B antibodies times the value for the titer of the anti B antibodies.

**19.** Procedure for the determination according to claim 3, in which three subsets of apolipoproteins belonging respectively to three groups of lipoprotein particles are determined, the first subset being constituted by the apolipoprotein B, belonging to the lipoprotein particles LPB constituting the first group, the second subset being constituted by the apolipoprotein E, belonging to the lipoprotein particles LPB:E constituting the second group, and the third subset being constituted by the apolipoproteins A I, A II and C III belonging to the lipoprotein particles LPE:CI:CII: CIII:B, LPB:CIII, LPE: AII, LPAI:E, LPA I and LP AI:AII, constituting the third group, characterized in that the medium containing the three subsets to be determined is placed respectively in the presence of a mixture of antibodies directed against the apolipoprotein C III (anti C III antibodies), against the apolipoprotein A II (anti A II antibodies), against the apolipoprotein A I, against the apolipoprotein E (anti E antibodies) and against the apolipoprotein B (anti B antibodies), the anti C III, anti A II and anti A I antibodies being in excess with respect to the anti E and anti B antibodies respectively, and the anti E antibodies being in excess with respect to the anti B antibodies.

**20.** In vitro screening procedure for diseases, in particular diseases correlated with contents of apolipoproteins lying outside the zone delimited by the extreme value usually corresponding to the physiological state of a healthy human being or animal, by using the determination process according to anyone of claims 3 to 19.

**21.** Support, in particular plate or film covered with gel ready for use in the implementation of the determination procedure or screening procedure according to Claims 3 to 20, characterized in that it consists in a mixture of antibodies corresponding to the generation of the precipitation zone of the lipoprotein particles carrying the apolipoproteins to be determined.

**22.** Determination kit containing a support, in particular a plate or film covered with gel, according to Claim 21, and a least a standard solution of defined dilution, previously set up from standard samples containing determined amounts of apolipoprotein sub-sets, which are to be titrated in a biological medium, or preferably a standard biological solution of preferably several determined dilutions, advantageously 3 or 4 determined dilutions, containing a known amount of apolipoprotein sub-sets which are to be titrated in a biological medium.

## Patentansprüche

**1.** Verfahren zur simultanen Bestimmung des Gehaltes an jeder von zwei oder mehreren Gruppen von unterschiedlichen und unabhängigen Teilchen in einem Teilchengemisch mittels differentieller Immunodiffusion der genannten Teilchengruppen an einem Gel, wobei die Teilchengruppen folgendes enthalten:

1. eine erste Gruppe von Teilchen, die jeweils ein erstes Antigen aufweisen und
2. mindestens eine zweite Gruppe von Teilchen, die jeweils ein zweites Antigen aufweisen und von denen mindestens einige das genannte erste Antigen tragen, wobei das genannte zweite Antigen nicht von den Teilchen der ersten Gruppe getragen wird,

mit den folgenden Verfahrensschritten:

- Behandlung des Teilchengemisches mit einem Gemisch von Antikörpern, welches einen ersten, gegen das erste Antigen gerichteten Antikörper und einen zweiten, gegen das zweite Antigen gerichteten Antikörper umfaßt, wobei das Antikörpergemisch den ersten Antikörper mindestens in einer zur Ausfällung der ersten Gruppe in dem Teilchengemisch ausreichenden Menge enthält, und wobei das Antikörpergemisch den zweiten Antikörper mindestens in einer zur Ausfällung der zweiten Gruppe in dem Teilchengemisch ausreichenden Menge und im Überschuß zu der genannten Menge des ersten Antikörpers enthält, so daß der jeweilige Gehalt an der jeweils ersten und zweiten Gruppe in dem genannten Teilchengemisch bestimmt werden kann.

**2.** Kit zur simultanen Bestimmung des Gehaltes an jeder von zwei oder mehreren Gruppen von unterschiedlichen und unabhängigen, Antigene tragenden Teilchen in einem Gemisch von Antigenen tragenden, im Anspruch 1 definierten Teilchen, der eine Gelplatte und ein vorbestimmte Mengen der die ersten und zweiten Antigene aufweisenden Teilchen enthaltendes Gemisch umfaßt.

**3.** Verfahren zur simultanen Bestimmung durch differentielle Immunodiffusion an einem plattenförmigen Gel von mindestens drei Unterensembles von Apolipoproteinen, die jeweils zu Gruppen von in einem biologischen Medium enthaltenen Lipoproteinteilchen gehören, wobei diese drei Gruppen von Lipoproteinteilchen so beschaffen sind, daß sie ein "gemeinsames Apolipoprotein" einschließen und wie folgt definierbar sind:

- eine der im folgenden mit erste bezeichneten Gruppen ist von Lipoproteinteilchen gebildet, die sämtlich das vorgenannte "gemeinsame Apolipoprotein" und gegebenenfalls andere Apolipoproteine $X_m$ aufweisen, wobei das "gemeinsame Apolipoprotein" von sämtlichen der Lipoproteinteilchen der das erste Unterensemble bildenden ersten Gruppe getragen wird,
- eine weitere, im folgenden mit zweite Gruppe oder intermediäre Gruppe bezeichnete Gruppe, die so beschaffen ist,
  - daß sie entweder bevorzugt von sämtlich das vorgenannte "gemeinsame Apolipoprotein" tragenden Lipoproteinteilchen gebildet ist und darüber hinaus mindestens ein Apolipoprotein $Y_n$ aufweist, das sich von den gegebenenfalls vorhandenen, von den Lipoproteinteilchen der ersten Gruppe getragenen, weiteren Apolipoproteinen unterscheidet, wobei dieses bzw. diese Apolipoprotein(e) $Y_n$ das zweite oder intermediäre Unterensemble bildet bzw. bilden,
  - oder von Lipoproteinteilchen gebildet ist, von denen einerseits ein Teil das vorgenannte, "gemeinsame Apolipoprotein" aufweist und das erste Unterensemble bildet sowie darüber hinaus mindestens ein Apolipoprotein $Z_n$ aufweist, das sich von den gegebenenfalls vorhandenen, anderen Apolipoproteinen $X_m$, die von den Lipoproteinteilchen der ersten Gruppe getragen werden, unterscheidet, und von denen andererseits der Rest der Lipoproteinteilchen das vorgenannte "gemeinsame Apolipoprotein" nicht aufweist, jedoch mindestens eines der Apolipoproteine $Z_p$ trägt, die von dem Teil der zuvor definierten Lipoproteinteilchen getragen werden und sich von den gegebenenfalls vorhandenen anderen Apolipoproteinen $X_m$, die von der ersten Gruppe der Lipoproteinteilchen getragen werden, unterscheiden, wobei dieses bzw. diese Apolipoprotein(e) $Z_n$ das zweite oder intermediäre Unterensemble bildet bzw. bilden,
- eine dritte oder letzte Gruppe, die wie folgt beschaffen ist:
  - sie ist entweder von Lipoproteingruppen gebildet, die sämtlich das oben definierte "gemeinsame Apolipoprotein" und darüber hinaus mindestens ein Apolipoprotein $W_q$ enthalten, welches sich einerseits von gegebenenfalls vorhandenen, von den Lipoproteinteilchen der ersten Gruppe getragenen Apolipoproteinen $X_m$ und sich andererseits von den von den Lipoproteinteilchen der zweiten Gruppe getragenen Apolipoproteinen unterscheidet, wobei das bzw. die Apolipoprotein(e) $W_q$ das dritte oder letzte Unterensemble bildet bzw. bilden;

- oder sie ist von Lipoproteinteilchen gebildet, von denen einerseits ein Teil das vorgenannte "gemeinsame Apolipoprotein" und darüber hinaus mindestens ein Apolipoprotein $T_1$ aufweist, welches sich gleichzeitig von den gegebenenfalls vorhandenen weiteren, von den Lipoproteinteilchen der ersten Gruppe getragenen Apolipoproteinen $X_m$ und den von den Lipoproteinteilchen der zweiten Gruppe getragenen Apolipoproteinen unterscheidet, und von denen andererseits der Rest der Lipoproteinteilchen nicht das vorgenannte "gemeinsame Apolipoprotein", sondern mindestens eines der von dem Teil der oben definierten Lipoproteinteilchen getragenen Apolipoproteine $T_1$ aufweist, wobei dieses bzw. diese Apolipoproteine $T_1$ das dritte oder letzte Unterensemble bildet bzw. bilden;

dadurch gekennzeichnet, daß

- man das die zu bestimmenden Unterensembles enthaltende biologische Medium mit einem Antikörpergemisch zusammenbringt, das
  . gegen das oder jedes der das dritte oder letzte Unterensemble bildende(n) Apolipoprotein(e) gerichtete Antikörper, und zwar in einer für die Ausfällung der Lipoproteine der dritten oder letzten Gruppe ausreichenden Menge;
  . gegen das oder jedes der das zweite oder intermediäre Unterensemble bildende(n) Apolipoprotein(e) gerichtete Antikörper, und zwar in einer für die Ausfällung der Lipoproteinteilchen der zweiten oder intermediären Gruppe ausreichenden Menge;
  . gegen das bei den Unterensembles vorhandene und das erste Unterensemble bildende "gemeinsame Apolipoprotein" gerichtete Antikörper, und zwar in einer für die Ausfällung der Lipoproteinteilchen der ersten Gruppe ausreichenden Menge,
  enthält;
- wobei die für die Ausfällung der Lipoproteinteilchen der dritten oder letzten Gruppe erforderliche Menge der Antikörper im Überschuß gegenüber der für die Ausfällung der Lipoproteinteilchen der zweiten oder intermediären Gruppe erforderlichen Menge der Antikörper vorhanden ist, und wobei die letztgenannte, für die Ausfällung der Lipoproteinteilchen der zweiten oder intermediären Gruppe erforderliche Menge der obengenannten Antikörper ihrerseits im Überschuß gegenüber der für die Ausfällung der das erste Unterensemble bildende "gemeinsame Apolipoprotein" tragenden Lipoproteinteilchen erforderlichen Menge der Antikörper vorhanden ist, und zwar in einem Verhältnis, daß man nach der Immunodiffusion drei diskrete Fronten erhält, wobei die Immunodiffusionsfront, die sich am nächsten zu dem Auftragsort des die zu bestimmenden Unterensembles enthaltenden Mediums befindet, der Ausfällung der Lipoproteinteilchen der dritten oder letzten Gruppe entspricht, die Immunodiffusionsfront, die sich am weitesten von dem genannten Auftragsort befindet, der Ausfällung der Lipoproteinteilchen der ersten Gruppe entspricht, und die intermediäre Immunodiffusionsfront zwischen den obengenannten nächsten und entferntesten Fronten der Ausfällung der Lipoproteinteilchen der zweiten Gruppe entspricht;
- und daß man die jeweils maximalen Abstände zwischen dem Auftragsort und jeder der drei Immunodiffusionsfronten mißt und jeden der Abstände mit denjenigen bei einem Eichmedium erhaltenen vergleicht, das bekannte Mengen an Apolipoproteinen der jeweiligen drei Unterensembles enthält.

**4.** Verfahren nach Anspruch 3 zur simultanen Bestimmung durch differentielle Immunodiffusion an einem plattenförmigen Gel von mindestens zwei Unterensembles von Apolipoproteinen, die jeweils zu mindestens zwei Gruppen von in einem biologischen Medium enthaltenen Lipoproteinteilchen gehören, wobei diese zwei Gruppen von Lipoproteinen so beschaffen sind, daß sie ein "gemeinsames Apolipoprotein" enthalten, und wobei

- eine der im folgenden mit erste Gruppe bezeichneten Gruppen von Lipoproteinteilchen gebildet ist, die sämtlich das vorgenannte "gemeinsame Apolipoprotein" und gegebenenfalls andere Apolipoproteine $X_m$ aufweisen, wobei dieses gemeinsame Apolipoprotein, das von sämtlichen der Lipoproteinteilchen der ersten Gruppe getragen wird, das erste Unterensemble bildet, und
- die andere Gruppe, die im folgenden mit zweite Gruppe bezeichnet wird, so beschaffen ist, daß sie
  - entweder von Lipoproteinteilchen gebildet ist, die sämtlich das oben definierte "gemeinsame Apolipoprotein" enthalten und darüber hinaus mindestens ein Apolipoprotein $Y_n$ aufweisen, das sich von den gegebenenfalls vorhandenen anderen Apolipoproteinen $X_m$, welche von den Lipoproteinteilchen der ersten Gruppe getragen werden, unterscheidet, wobei dieses bzw. diese Apolipoprotein(e) $Y_n$ das zweite Unterensemble bildet bzw. bilden;

- oder von Lipoproteinteilchen gebildet ist, von denen einerseits ein Teil das obengenannte "gemeinsame Apolipoprotein", das das erste Unterensemble bildet, und darüber hinaus mindestens ein Apolipoprotein $Z_p$ aufweist, das sich von den gegebenenfalls vorhandenen anderen Apolipoproteinen $X_m$, die von den Lipoproteinteilchen der ersten Gruppe getragen werden, unterscheidet, und von denen andererseits der Rest der Lipoproteinteilchen nicht das obengenannte "gemeinsame Apolipoprotein", das das erste Unterensemble bildet, sondern mindestens eines der Apolipoproteine $Z_p$ aufweist, die von dem Teil der oben definierten Lipoproteinteilchen getragen werden und sich von den gegebenenfalls vorhandenen anderen Apolipoproteinen $X_m$, die von der ersten Gruppe der Lipoproteinteilchen getragen werden, unterscheidet, wobei dieses bzw. diese Apolipoprotein(e) $Z_p$ das zweite Unterensemble bilden, dadurch gekennzeichnet, daß man
- das die zwei zu bestimmenden Unterensembles von Apolipoprotein enthaltende biologische Medium mit einem Gemisch von Antikörpern zusammenbringt, das
  . einerseits Antikörper enthält, die gegen das oder jedes der das zweite Unterensemble ($Y_n$ oder $Z_p$) bildenden Apolipoproteine gerichtet sind, und zwar in einer zur Ausfällung der Lipoproteinteilchen der zweiten Gruppe ausreichenden Menge;
  . andererseits Antikörper enthält, die gegen das vorgenannte "gemeinsame Apolipoprotein", das das erste Unterensemble bildet, gerichtet sind, und zwar in einer zur Ausfällung der Lipoproteinteilchen der ersten Gruppe ausreichenden Menge;
- wobei die Menge der für die Ausfällung der Lipoproteinteilchen der zweiten Gruppe erforderlichen Antikörper im Überschuß gegenüber der für die Ausfällung der Lipoproteinteilchen der ersten Gruppe erforderlichen Menge der vorgenannten Antikörper vorhanden ist, und zwar in einem Verhältnis, daß man nach der Immunodiffusion zwei diskrete Fronten enthält, wobei die Immunodiffusionsfront, die sich am nächsten zu dem Auftragsort des die zwei zu bestimmenden Unterensembles enthaltenden biologischen Mediums befindet, der Ausfällung der Lipoproteinteilchen der zweiten Gruppe und die Immunodiffusionsfront, die sich am entferntesten von dem genannten Auftragsort befindet, der Ausfällung der Lipoproteinteilchen der ersten Gruppe entspricht, und wobei die zwei Fronten jeweils identisch sind mit denjenigen, die man unter Bedingungen erhält, unter denen man nach dem Trennen der zwei Gruppen von Lipoproteinteilchen, welche das erste bzw. das zweite Unterensemble von Apolipoproteinen enthalten, einerseits die erste Gruppe der Lipoproteinteilchen mit gegen das gemeinsame Apolipoprotein, das das erste, oben definierte Unterensemble bildet, gerichteten Antikörpern zusammenbringt, und zwar in der gleichen Menge wie die oben für die Ausfällung der Lipoproteinteilchen der ersten, nicht von dem biologischen Medium getrennten und das das erste Unterensemble bildende Apolipoprotein tragenden Gruppe, und andererseits die zweite Gruppe von Lipoproteinteilchen mit gegen die Apolipoproteine, die das zweite oben definierte Unterensemble bilden, gerichteten Antikörpern zusammenbringt, und zwar in der gleichen Menge wie die oben für die Ausfällung der zweiten Gruppe der Lipoproteinteilchen, die nicht von dem biologischen Medium getrennt wurden und die die das zweite Unterensemble bildenden Apolipoproteine tragen.
- und daß man die jeweiligen maximalen Abstände zwischen dem Auftragsort und jeder der zwei Immunodiffusionsfronten mißt und jeden der Abstände in bezug auf diejenigen vergleicht, die mit einem biologischen Vergleichsmedium erhalten werden, das bekannte Mengen an den jeweiligen Apolipoproteinen der zwei Unterensembles enthält.

**5.** Verfahren nach Anspruch 3 zur simultanen Bestimmung durch differentielle Immunodiffusion an einem plattenförmigen Gel von zwei Unterensembles von Apolipoprotein, die jeweils zu zwei Gruppen von Lipoproteinteilchen gehören, welche in dem biologischen Medium enthalten sind, wobei die zwei Gruppen von Lipoproteinteilchen so beschaffen sind, daß sie ein "gemeinsames Apolipoprotein" enthalten,

- wobei eine der Gruppen, die nachfolgend mit erste Gruppe bezeichnet wird, aus Lipoproteinteilchen gebildet ist, die sämtlich das obengenannte "gemeinsame Apolipoprotein" aufweisen und gegebenenfalls andere Apolipoproteine $X_m$ tragen, wobei das "gemeinsame Apolipoprotein" von sämtlichen Lipoproteinteilchen der ersten Gruppe getragen wird und das erste Unterensemble bildet, und die andere Gruppe, die im folgenden mit zweite Gruppe bezeichnet wird, so beschaffen ist, daß sie:
  - entweder von Lipoproteinteilchen gebildet ist, die sämtlich das oben definierte "gemeinsame Apolipoprotein" enthalten und darüber hinaus mindestens ein Apolipoprotein $Y_n$ aufweisen, das

sich von den gegebenenfalls vorhandenen anderen Apolipoproteinen $X_m$, welche von den Lipoproteinteilchen der ersten Gruppe getragen werden, unterscheidet, wobei dieses bzw. diese Apolipoprotein(e) $Y_n$ das zweite Unterensemble bildet bzw. bilden;

- oder von Lipoproteinteilchen gebildet ist, von denen einerseits ein Teil das obengenannte "gemeinsame Apolipoprotein" der ersten Gruppe und darüber hinaus mindestens ein Apolipoprotein $Z_p$ aufweist, das sich von den gegebenenfalls vorhandenen anderen Apolipoproteinen $X_m$, die von den Lipoproteinteilchen der ersten Gruppe getragen werden, unterscheidet, und von denen andererseits der Rest der Lipoproteinteilchen nicht das obengenannte "gemeinsame Apolipoprotein", das das erste Unterensemble bildet, sondern mindestens eines der Apolipoproteine $Z_p$ aufweist, die von dem Teil der oben definierten Lipoproteinteilchen getragen werden und sich von den gegebenenfalls vorhandenen anderen Apolipoproteinen $X_m$, die von der ersten Gruppe der Lipoproteinteilchen getragen werden, unterscheidet, wobei dieses bzw. diese Apolipoprotein(e) $Z_p$ das zweite Unterensemble bilden,

dadurch gekennzeichnet, daß man

- das die zwei zu bestimmenden Unterensembles von Apolipoprotein enthaltende biologische Medium mit einem Gemisch von Antikörpern zusammenbringt, das
  . einerseits Antikörper enthält, die gegen das oder jedes der Apolipoproteine des zweiten Unterensembles ($Y_n$ oder $Z_p$) gerichtet sind, und zwar in einer zur Ausfällung der Lipoproteinteilchen der zweiten Gruppe ausreichenden Menge;
  . andererseits Antikörper enthält, die gegen das vorgenannte "gemeinsame Apolipoprotein", der zwei Unterensembles gerichtet sind, und zwar in einer zur Ausfällung der Lipoproteinteilchen der ersten Gruppe ausreichenden Menge;
- wobei die Menge der für die Ausfällung der Lipoproteinteilchen der zweiten Gruppe erforderlichen Antikörper im Überschuß gegenüber der für die Ausfällung der Lipoproteinteilchen der ersten Gruppe erforderlichen Menge der vorgenannten Antikörper vorhanden ist, und zwar in einem Verhältnis, daß man nach der Immunodiffusion zwei diskrete Fronten enthält, wobei die Immunodiffusionsfront, die sich am nächsten zu dem Auftragsort des die zwei zu bestimmenden Unterensembles enthaltenden biologischen Mediums befindet, der Ausfällung der Lipoproteinteilchen der zweiten Gruppe und die Immunodiffusionsfront, die sich am entferntesten von dem genannten Auftragsort befindet, der Ausfällung der Lipoproteinteilchen der ersten Gruppe entspricht, und wobei die zwei Fronten jeweils identisch sind mit denjenigen, die man unter Bedingungen erhält, unter denen man nach dem Trennen der zwei Gruppen von Lipoproteinteilchen, welche das erste bzw. das zweite Unterensemble von Apolipoproteinen enthalten, einerseits die erste Gruppe der Lipoproteinteilchen mit gegen das gemeinsame Apolipoprotein der ersten Gruppe gerichteten Antikörpern zusammenbringt, und zwar in der gleichen Menge wie die oben für die Ausfällung der nicht von dem biologischen Medium getrennten und das Apolipoprotein des ersten Unterensembles tragenden Lipoproteinteilchen, und daß man andererseits die zweite Gruppe von Lipoproteinteilchen in Kontakt mit den gegen das bzw. die Apolipoprotein(e) des zweiten Unterensembles bringt, das bzw. die sich von dem bzw. den Apolipoprotein(en) des ersten Unterensembles und von den gegebenenfalls vorhandenen anderen, von den Lipoproteinteilchen der ersten Gruppe getragenen Apolipoproteinen unterscheidet bzw. unterscheiden, und zwar in der gleichen Menge zu der oben verwendeten, um die nicht aus dem biologischen Medium abgetrennten Lipoproteinteilchen, die die Apolipoproteine des zweiten Unterensembles tragen, auszufällen,
- und daß man die jeweiligen maximalen Abstände zwischen dem Auftragsort und jeder der zwei Immunodiffusionsfronten mißt und jeden der Abstände in bezug auf diejenigen vergleicht, die mit einem biologischen Vergleichsmedium erhalten werden, das bekannte Mengen an den jeweiligen Apolipoproteinen der zwei Unterensembles enthält.

**6.** Verfahren zur Bestimmung nach Anspruch 5, bei dem das Apolipoprotein des ersten Unterensembles zu den freien und identischen, die erste Gruppe bildenden Lipoproteinteilchen gehört.

**7.** Verfahren zur Bestimmung nach Anspruch 5, bei dem die erste Gruppe einerseits von freien und untereinander identischen Lipoproteinteilchen, die das das erste Unterensemble bildende gemeinsame Apolipoprotein aufweisen, und andererseits von komplexen Lipoproteinteilchen, die außer den obengenannten gemeinsamen Apolipoproteinen mindestens eines von den genannten gemeinsamen Apolipoproteinen und von den das zweite Unterensemble bildenden Apolipoproteinen verschiedenes Apolipoprotein aufweisen, gebildet ist.

8. Verfahren zur Bestimmung nach einem beliebigen der Ansprüche 5 bis 7, bei dem das zweite Unterensemble von einem Apolipoprotein gebildet ist, das von dem das erste Unterensemble bildenden gemeinsamen Apolipoprotein und von gegebenenfalls vorhandenen anderen, von den Lipoproteinteilchen der ersten Gruppe getragenen Apolipoproteinen verschieden ist, wobei das genannte Apolipoprotein von den komplexen Lipoproteinteilchen getragen wird, die darüber hinaus sämtliche das erste Unterensemble bildenden Apolipoproteine aufweisen.

9. Verfahren zur Bestimmung nach einem beliebigen der Ansprüche 5 bis 7, bei dem das zweite Unterensemble von mindestens einem Apolipoprotein gebildet wird, das sich von dem das erste Unterensemble bildenden und von den gegebenenfalls vorhandenen anderen, von den Lipoproteinteilchen der ersten Gruppe getragenen Apolipoproteinen unterscheidet, wobei das genannte Apolipoprotein einerseits von komplexen Teilchen, die darüber hinaus das das erste Unterensemble bildende Apolipoprotein aufweisen, und andererseits von einfachen oder komplexen Lipoproteinteilchen, welche nicht das das erste Unterensemble bildende Apolipoprotein aufweisen, getragen wird.

10. Verfahren zur Bestimmung nach einem beliebigen der Ansprüche 3 bis 9, bei dem die differentielle Immunodiffusion als Elektroimmunodiffusion nach der Methode von Laurell durchgeführt wird, wodurch mindestens drei Peaks erhalten werden.

11. Verfahren zur Bestimmung nach einem beliebigen der Ansprüche 3 bis 9 durch Radialimmunodiffusion nach der Methode von Mancini, wodurch mindestens drei Ringe erhalten werden.

12. Verfahren zur Bestimmung nach einem beliebigen der Ansprüche 5 bis 11, bei dem die Mindestmenge der einzusetzenden, gegen das bzw. die Apolipoprotein(e) des zweiten Unterensembles gerichteten Antikörper von dem Erscheinen von zwei diskreten Immunodiffusionsfronten an bestimmt wird und bevorzugt in einem Bereich liegt, dessen Minimalwert von dem Erscheinen der zwei diskreten Immunodiffusionsfronten und dessen Maximalwert bestimmt wird, sobald die Lipoproteinteilchen, die die Apolipoproteine des zweiten Unterensembles tragen, nicht mehr diffundieren und an dem Auftragsort des biologischen Mediums ausgefällt werden, wobei die der Ausfällung der die Apolipoproteine des ersten Unterensembles tragenden Lipoproteinteilchen entsprechende Immunodiffusionsfront nicht verändert wird, wenn die Menge der gegen das bzw. die Apolipoproteine des zweiten Unterensembles gerichteten Antikörper größer ist als der oben definierte Minimalwert.

13. Verfahren zur Bestimmung nach einem beliebigen der Ansprüche 5 bis 12, bei dem die Menge der gegen das bzw. die Apolipoprotein(e) des zweiten Unterensembles gerichteten Antikörper so bemessen ist, daß der maximale Abstand zwischen dem Auftragsort und der Immunodiffusionsfront, die der Ausfällung der die Apolipoproteine des zweiten Unterensembles enthaltenden Lipoproteinteilchen entspricht, zwischen etwa 1/2 bis etwa 1/8, vorzugsweise zwischen etwa 1/3 bis etwa 1/4, des maximalen Abstandes zwischen dem Auftragsort und der der Ausfällung der die Apolipoproteine des ersten Unterensembles enthaltenden Lipoproteinteilchen entsprechenden Immunodiffusionsfront beträgt.

14. Verfahren zur Bestimmung nach einem beliebigen der Ansprüche 5 bis 13, bei dem die Eichung ausgehend von einem Medium durchgeführt wird, in dem das erste Unterensemble eine bekannte Menge (A) an dem beiden Gruppen gemeinsamen Apolipoprotein und das zweite Unterensemble eine bzw. mehrere bekannte Menge(n) $(B)_i$ von Apolipoprotein(en), das bzw. die sich von dem den zwei Gruppen gemeinsamen Apolipoprotein sowie von den gegebenenfalls vorhandenen anderen, zu den Lipoproteinteilchen der ersten Gruppe gehörenden Apolipoproteinen unterscheidet bzw. unterscheiden, enthält,

- indem man auf die Platte gegen jedes der Apolipoproteine des zweiten Unterensembles gerichtete Antikörper aufbringt, und zwar in einer für die Ausfällung der Lipoproteinteilchen der zweiten Gruppe erforderlichen Menge, wobei jede dieser Mengen jeweils im Überschuß, bezogen auf zur Ausfällung der Lipoproteinteilchen der ersten Gruppe erforderliche Antikörper, eingesetzt wird, wobei der Überschuß der gegen die Apolipoproteine des zweiten Unterensembles gerichteten Antikörper, bezogen auf gegen das Apolipoprotein des ersten Unterensembles gerichtete Antikörper, durch die folgende Beziehung ausgedrückt werden kann:

$$V_{2i}T_{2i} \times \frac{B_i}{A} \text{ größer als } V_1T_1$$

in der $V_1$ und $T_1$ jeweils das Volumen und den Titer der gegen das Apolipoprotein des ersten Unterensembles sowie $V_{2i}$ und $T_{2i}$ jeweils die Volumina und die Titer der jeweils gegen jedes der Apolipoproteine des zweiten Unterensembles gerichteten Antikörper bedeuten.

**15.** Verfahren zur Bestimmung durch Elektroimmunodiffusion gemäß einem beliebigen der Ansprüche 5 bis 10 und 12 bis 14, bei dem man bestimmt:

- die Menge von Antikörpern $V_1$, die zur Ausfällung der das Apolipoprotein des ersten Unterensembles tragenden Lipoproteinteilchen bestimmt sind, wobei das genannte Apolipoprotein beiden Gruppen gemeinsam ist, damit die Höhe des erhaltenen Peaks $H_1$ ist, indem man auf die Platte eine bekannte Menge der Antikörper $v_1$ entsprechend der Ausfällung der Lipoproteinteilchen der ersten Gruppe aufträgt, was eine Höhe $h_1$ ergibt, wobei die Menge $V_1$ anschließend nach der folgenden Formel berechnet wird:

$$V_1 = x \,\% \, \frac{h_1 v_1}{H_1}$$

wobei x % den Prozentsatz des den zwei Gruppen gemeinsamen, in dem ersten Unterensemble enthaltenen Apolipoproteins bedeutet, bezogen auf die Gesamtmenge dieses in dem Medium enthaltenen Apolipoproteins, und daß man bestimmt:

- die Menge der Antikörper $V_2$, die zur Ausfällung der das bzw. die Apolipoprotein(e) des zweiten Unterensembles, die verschieden von den den zwei Gruppen gemeinsamen und verschieden von gegebenenfalls vorhandenen anderen, von den Lipoproteinteilchen der ersten Gruppe getragenen Apolipoproteinen sind, tragenden Lipoproteinteilchen bestimmt ist, damit die Höhe des erhaltenen Peaks $H_2$ ist, indem man auf die Platte eine bekannte Menge von Antikörpern $v_2$ entsprechend der Ausfällung der Lipoproteinteilchen der zweiten Gruppe aufträgt, was eine Höhe $h_2$ ergibt, wobei die Menge $V_2$ anschließend nach der folgenden Formel berechnet wird:

$$V_2 = \frac{h_2 v_2}{H_2}$$

**16.** Verfahren zur Bestimmung durch Radialimmunodiffusion gemäß einem beliebigen der Ansprüche 5 bis 9 und 11 bis 14, in dem man bestimmt:

- die Menge der Antikörper $V_1$, die zur Ausfällung der Lipoproteinteilchen bestimmt ist, welche das Apolipoprotein des ersten Unterensembles tragen, wobei dieses Apolipoprotein beiden Gruppen gemeinsam ist, damit der Durchmesser des erhaltenen Ringes $D_1$ ist, indem man auf die Platte eine bekannte Menge der Antikörper $v_1$, entsprechend der Ausfällung der Lipoproteinteilchen der ersten Gruppe, aufbringt, was einen Durchmesser $d_1$ ergibt, wobei anschließend die Menge $V_1$ gemäß der folgenden Formel

$$V_1 = x \,\% \, \frac{d_1^2}{D_1^2} \, v_1$$

berechnet wird, wobei x % den Prozentsatz des den zwei Gruppen gemeinsamen und in dem ersten Unterensemble enthaltenen Apolipoproteins bedeutet, bezogen auf die Gesamtmenge dieses in dem Medium enthaltenden Apolipoproteins,

- die Menge der Antikörper $V_2$, die zur Ausfällung der Lipoproteinteilchen, welche das bzw. die Apolipoprotein(e) des zweiten Unterensembles tragen, die verschieden von dem den zwei Gruppen gemeinsamen Apolipoprotein und verschieden von gegebenenfalls vorhandenen anderen, von den Lipoproteinteilchen der ersten Gruppe getragenen Apolipoproteinen sind, bestimmt sind, damit der Durchmesser des erhaltenen Ringes $D_2$ ist, wird bestimmt, indem man auf die Platte eine bekannte Menge von Antikörpern $v_2$ aufbringt, die der Ausfällung der Apolipoproteine des zweiten Unterensembles entspricht, was einen Durchmesser $d_2$ ergibt, und anschließend die Menge $V_2$ nach der folgenden Formel

$$V_2 = \frac{d_2^2 v_2^2}{D_2^2}$$

berechnet wird.

**17.** Verfahren zur Bestimmung gemäß einem beliebigen der Ansprüche 5 bis 16, bei dem das erste Unterensemble von dem Apolipoprotein AI gebildet wird, das von den die erste Gruppe bildenden Lipoproteinteilchen LP AI und LP AI:E getragen wird, und das zweite Unterensemble von dem Apolipoprotein AII gebildet wird, das von den die zweite Gruppe bildenden Lipoproteinteilchen LP AI:AII und LPE:AII getragen wird, dadurch gekennzeichnet, daß man das die zwei zu bestimmenden Unterensembles enthaltende Medium mit einem Gemisch von Antikörpern zusammenbringt, die jeweils gegen das Apolipoprotein AII (Antikörper anti-AII) und gegen das Apolipoprotein AI (Antikörper anti-AI) gerichtet sind, und zwar unter solchen Bedingungen, daß die gegen das Apolipoprotein AII gerichteten Antikörper im Überschuß, bezogen auf die gegen das Apolipoprotein AI gerichteten Antikörper, vorliegen, wobei dieser Überschuß insbesondere der Tatsache entspricht, daß das Produkt des Wertes des Volumens der auf die Platte aufgetragenen Antikörper anti-AII durch den Wert des Titers der Antikörper anti-AII größer ist als das Produkt des Wertes des Volumens der auf die Platte aufgetragenen Antikörper anti-AI durch den Wert des Titers der Antikörper anti-AI.

**18.** Verfahren zur Bestimmung nach einem beliebigen der Ansprüche 5 bis 16, bei dem das erste Unterensemble von dem Apolipoprotein B gebildet wird, das von den die erste Gruppe bildenden Lipoproteinteilchen LPB getragen wird, und das zweite Unterensemble von den zwei Apolipoproteinen E und CIII gebildet wird, die von den die zweite Gruppe bildenden Lipoproteinteilchen LPB:E, LPB:CIII, LPE:CI:CII:CIII:B, LPAI:E und LPE:AII getragen werden, dadurch gekennzeichnet, daß man das die zwei zu bestimmenden Unterensemble enthaltende Medium mit einem Gemisch von Antikörpern zusammenbringt, die jeweils gegen das Apolipoprotein E (Antikörper anti-E) Apolipoprotein CIII (Antikörper anti-CIII) und Apolipoprotein B (Antikörper anti-B) gerichtet sind, und zwar unter solchen Bedingungen, daß die gegen das Apolipoprotein E gerichteten Antikörper im Überschuß, bezogen auf die gegen das Apolipoprotein B gerichteten Antikörper, und die gegen das Apolipoprotein CIII gerichteten Antikörper im Überschuß, bezogen auf die gegen das Apolipoprotein B gerichteten Antikörper vorliegen, wobei der Überschuß der Antikörper anti-CIII in bezug auf die Antikörper anti-B durch die Tatsache ausgedrückt werden kann, daß das Produkt des Wertes des Volumens der Antikörper anti-CIII durch den Wert des Titers der Antikörper anti-CIII größer als ein Sechstel des Produktes des Wertes des Volumens der Antikörper anti-B durch den Wert des Titers der Antikörper anti-B ist, und der Überschuß der Antikörper anti-E, bezogen auf Antikörper anti-B, durch die Tatsache ausgedrückt werden kann, daß das Produkt aus dem Wert des Volumens der Antikörper anti-E durch den Wert des Titers der Antikörper anti-E größer als ein Zwölftel des Produktes des Wertes des Volumens der Antikörper anti-B durch den Wert des Titers der Antikörper anti-B ist.

**19.** Verfahren zur Bestimmung nach Anspruch 3, bei dem man drei Unterensembles von Apolipoproteinen bestimmt, die jeweils zu drei Gruppen von Lipoproteinteilchen gehören, wobei das erste Unterensemble von dem zu den die erste Gruppe bildenden Lipoproteinteilchen LPB gehörenden Apolipoprotein B

gebildet wird, das zweite Unterensemble von dem zu den die zweite Gruppe bildenden Lipoproteinteilchen LPB:E gehörenden Apolipoprotein E gebildet wird und das dritte Unterensemble von den zu den die dritte Gruppe bildenden Lipoproteinteilchen LPE:CI:CII:CIII:B, LPB:CIII, LPE:AII; LPAI:E, LPAI und LPAI:AII gehörenden Apolipoproteinen AI, AII und CIII gebildet wird, dadurch gekennzeichnet, daß man das die drei zu bestimmenden Unterensembles enthaltende Medium jeweils mit einem Gemisch von Antikörpern zusammenbringt, die gegen das Apolipoprotein CIII (Antikörper anti-CIII), gegen das Apolipoprotein AII (Antikörper anti-AII), gegen das Apolipoprotein AI (Antikörper anti-AI), gegen das Apolipoprotein E (Antikörper anti-E) und gegen das Apolipoprotein B (Antikörper anti-B) gerichtet sind, wobei die Antikörper anti-CIII, anti-AII und anti-AI jeweils im Überschuß, bezogen auf die Antikörper anti-E, und anti-B und die Antikörper E im Überschuß, bezogen auf die Antikörper anti-B, vorliegen.

**20.** Verfahren zur in-vitro-Erkennung von Krankheiten, die insbesondere mit Apolipoproteinspiegeln korreliert sind, welche sich außerhalb eines durch allgemein einem physiologischen Zustand eines gesunden menschlichen Wesens oder Tieres entsprechende Extremwerte begrenzten Bereiches befinden, bei dem das Bestimmungsverfahren gemäß einem beliebigen der Ansprüche 3 bis 19 angewendet wird.

**21.** Träger, insbesondere Platte oder Film bzw. Folie, der mit einem Gel beschichtet und zur Verwendung in einem Bestimmungs- oder Erkennungsverfahren gemäß den Ansprüchen 3 bis 20 zugerichtet ist, dadurch gekennzeichnet, daß er ein Gemisch von Antikörpern umfaßt, die jeweils dem Erreichen eines Bereiches der Ausfällung von die zu bestimmenden Apolipoproteine tragenden Lipoproteinteilchen entsprechen.

**22.** Bestimmungskit oder -mittel, enthaltend einen Träger, insbesondere eine Gelplatte oder einen gelbeschichteten Film bzw. eine ebensolche Folie gemäß Anspruch 21 und mindestens eine Eichkurve, die vorher anhand von Eichproben mit einem Gehalt an vorbestimmten Mengen an Unterensembles von Apolipoproteinen, welche anderweitig in einem biologischen Medium zu bestimmen sind, festgelegt wurde, oder vorzugsweise eine biologische Eichlösung, bevorzugt mit mehreren vorbestimmten Verdünnungen, bevorzugt 3 oder 4 vorbestimmten Verdünnungen, enthaltend eine bekannte Menge von Unterensembles von anderweitig in einem biologischen Medium zu bestimmenden Apolipoproteinen.

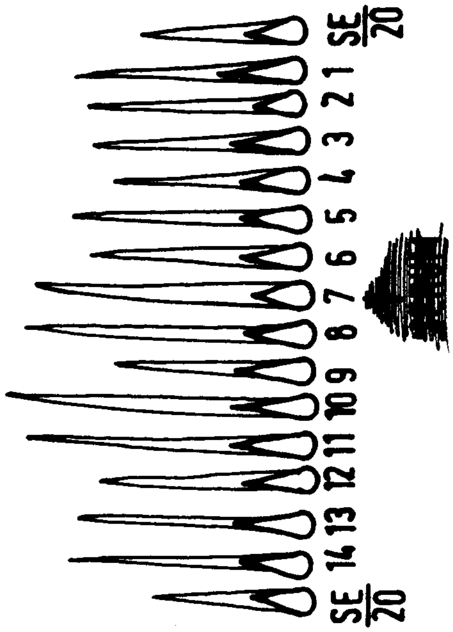
SE/20
14
13
12
11
10
9
8
7
6
5
4
3
2
1
SE/20

FIG. 2

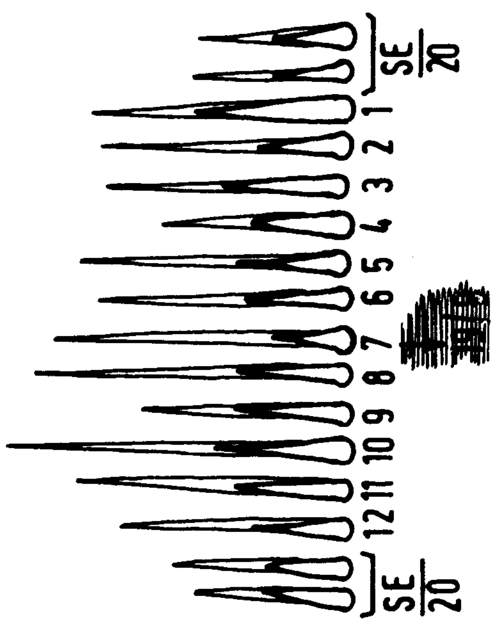
SE/20
12
11
10
9
8
7
6
5
4
3
2
1
SE/20

FIG. 1

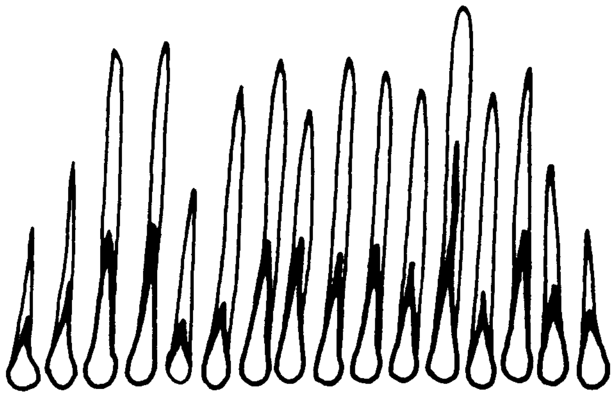

FIG.3

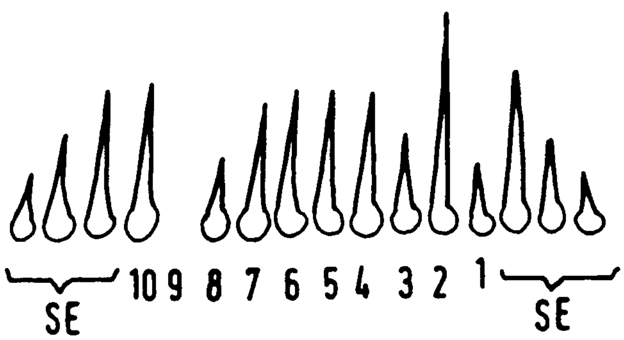


FIG.4

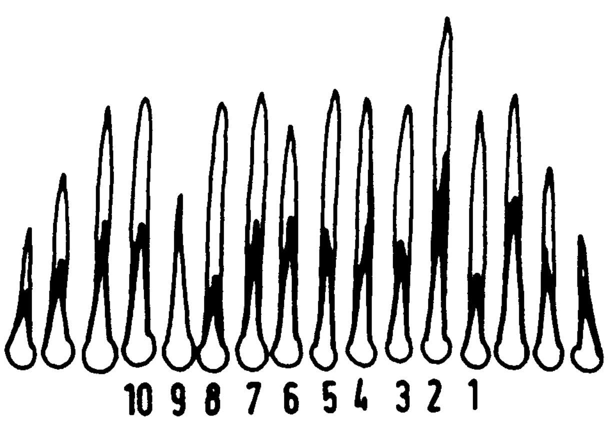


FIG.5

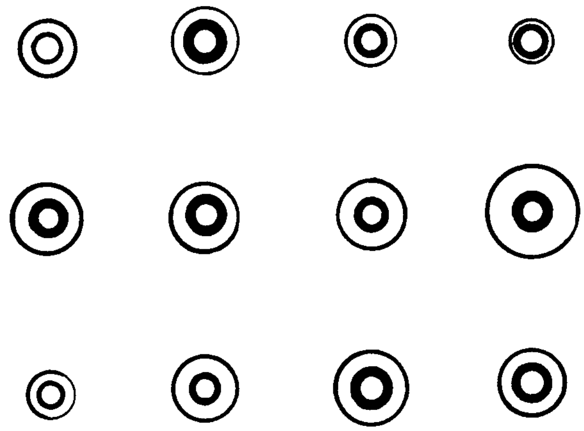

FIG.6

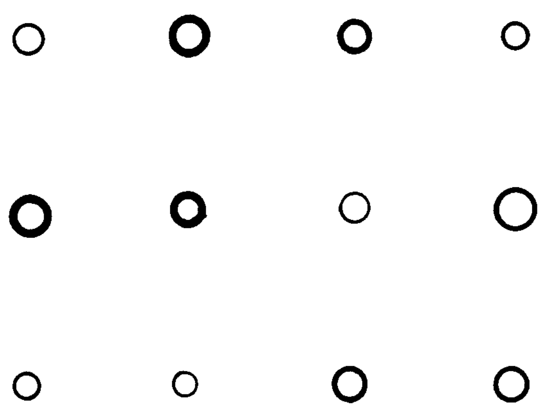

FIG.7

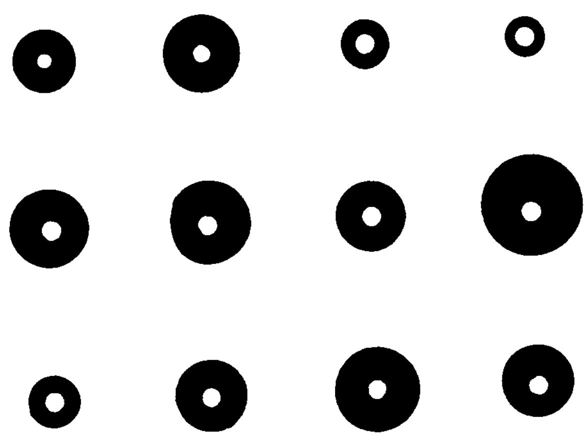

FIG.8